# EUROPEAN PATENT APPLICATION

(11) **EP 4 755 375 A1**
(43) Date of publication of application: **10.06.2026**
(21) Application number: 24847268.0
(22) Date of filing: 19.01.2024
(51) Int. Cl.: A61K 9/20, A61K 31/506, A61P 35/00, A61P 35/02, A61P 9/00, A61P 19/10, A61K 31/74

(54) **PHARMACEUTICAL COMPOSITION CONTAINING SUBSTITUTED INDOL-5-OL DERIVATIVE AND USE THEREOF**

(30) Priority: 03.08.2023 CN 202310973263
(71) Applicant: Changshan Conjuchem Biopharmaceutical Research and Development (Hebei)., Ltd., Shijiazhuang, Hebei 050000 (CN)
(72) Inventor: SUN, Xiaocui, Shijiazhuang, Hebei 050000 (CN); XU, Geng, Shijiazhuang, Hebei 050000 (CN); QIE, Zhenggang, Shijiazhuang, Hebei 050000 (CN); WANG, Yan, Shijiazhuang, Hebei 050000 (CN); TIAN, Yingtao, Shijiazhuang, Hebei 050000 (CN); LIU, Fengjie, Shijiazhuang, Hebei 050000 (CN); YAN, Yunxia, Shijiazhuang, Hebei 050000 (CN); SUN, Yungai, Shijiazhuang, Hebei 050000 (CN); MA, Chaohui, Shijiazhuang, Hebei 050000 (CN); WANG, Fei, Shijiazhuang, Hebei 050000 (CN); LIU, Xiaohui, Shijiazhuang, Hebei 050000 (CN); DONG, Linlin, Shijiazhuang, Hebei 050000 (CN); ZHANG, Zhe, Shijiazhuang, Hebei 050000 (CN)
(74) Representative: Forresters IP LLP
(86) International application number: PCT/CN2024/073184
(87) International publication number: WO 2025/025535

(57) **Abstract**

Disclosed is a first pharmaceutical composition comprising a substituted indol-5-ol derivative, characterized in that the first pharmaceutical composition contains (a) a therapeutically effective amount of a compound of formula (I) or a pharmaceutically acceptable salt thereof; and (b) one or more polymer materials, for example, one, two, three, four, or five types of polymer materials.

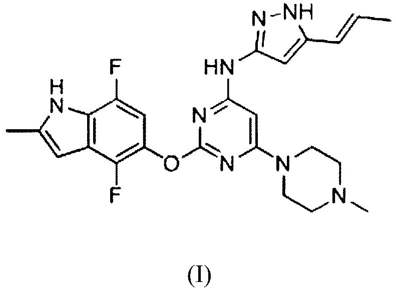

## Description

### TECHNICAL FIELD

The present invention relates to the technical field of pharmaceuticals, and specifically relates to a pharmaceutical composition containing a substituted indol-5-ol derivative and use thereof.

### BACKGROUND

The compound involved in the present invention is a substituted indol-5-ol derivative, which is a compound independently developed by the applicant for modulating protein kinases and treating protein kinase-mediated diseases, primarily serving as a multi-target broad-spectrum anticancer drug. For the relevant compounds related to the present invention, refer to the compound patents: ZL 201380069244.9 and ZL 201480026101.4, the relevant contents of which may be incorporated into the present patent application by reference.

To increase the efficiency of new drug discovery and research, the applicant has been dedicated to finding new methods for drug design and drug screening, and to conducting research on molecular design, synthesis, and anticancer activity of inhibitors. Small molecule antitumor drugs based on new pharmacological mechanisms are designed and synthesized for tumor-specific target proteins. The applicant has resolved the crystal structures of multiple protein kinases or protein kinase complexes, and has applied computer-aided design and fragment-based drug design methods to design new small molecule kinase inhibitors. Subsequently, the disease targets for treatment and the action targets are determined; through processes including model confirmation, hit compound discovery, lead compound identification, and lead compound optimization, an excellent compound, a candidate drug, is ultimately obtained. Subsequent development work is carried out, including further confirmation of drug targets, scale-up synthesis of the compound, process optimization, drug evaluation, and formulation development.

At present, no relevant reports on the pharmaceutical composition containing a substituted indol-5-ol derivative and use thereof as claimed in the present invention have been found in the prior art.

### CONTENT OF THE INVENTION

In view of this, the present invention provides a first pharmaceutical composition containing a substituted indol-5-ol derivative, the first pharmaceutical composition contains
(a) a therapeutically effective amount of a compound of formula (I), or a pharmaceutically acceptable salt thereof; and 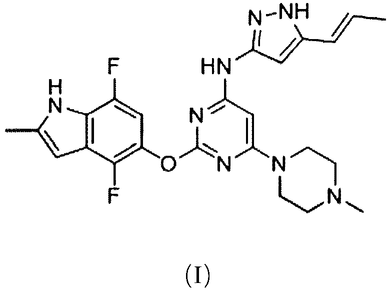
(b) one or more polymer materials, such as 1, 2, 3, 4, or 5 polymer materials.

Furthermore, the polymer material is selected from one or more of the following: hypromellose acetate succinate, hydroxyalkyl cellulose, povidone, copovidone, polypropylene resin, polyvinyl caprolactam-polyvinyl acetate-polyethylene glycol graft copolymer, and polyethylene glycol. Furthermore, the hypromellose acetate succinate is hypromellose acetate succinate AS-MMP and/or hypromellose acetate succinate AS-MF. Furthermore, the hydroxyalkyl cellulose is hydroxypropyl cellulose, hypromellose, and/or hydroxyethyl cellulose. Furthermore, the hydroxypropyl cellulose is hydroxypropyl cellulose EXF, hydroxypropyl cellulose HXF, and/or hydroxypropyl cellulose LXF. Furthermore, the hypromellose is hypromellose E3, hypromellose E5, and/or hypromellose E15. Furthermore, the povidone is povidone K25, povidone K30, povidone K-12, and/or povidone K-17. Furthermore, the copovidone is copovidone S630. Furthermore, the polypropylene resin is polypropylene resin E100. Furthermore, the polyethylene glycol is polyethylene glycol 4000 and/or polyethylene glycol 6000. Furthermore, the polymer material is povidone K30, hypromellose E3, hypromellose E15, hypromellose acetate succinate AS-MMP, hydroxypropyl cellulose EXF, copovidone S630, polyethylene glycol 4000, polyvinyl caprolactam-polyvinyl acetate-polyethylene glycol graft copolymer, or polypropylene resin E100. Furthermore, a mass ratio of the compound of formula (I) to the polymer material is 1:1 to 1:10. Furthermore, the mass ratio of the compound of formula (I) to the polymer material is 1:1 to 1:6. Furthermore, the mass ratio of the compound of formula (I) to the polymer material is 1:3 to 1:6.

Furthermore, the first pharmaceutical composition further contains one or more antioxidants, such as 1, 2, 3, 4, or 5 antioxidants. Furthermore, the antioxidant is selected from one or more of the following: butylated hydroxyanisole, butylated hydroxytoluene, sodium metabisulfite, vitamin E, vitamin E acetate, and L-ascorbyl palmitate. Furthermore, the antioxidant is sodium metabisulfite. Furthermore, the antioxidant is present in an amount of 0.01% to 3% such as about 0.2% based on a weight of the first pharmaceutical composition.

Furthermore, the first pharmaceutical composition is in a form of a solid dispersion.

According to one aspect of the present invention, the present invention provides a second pharmaceutical composition comprising the aforementioned first pharmaceutical composition, the second pharmaceutical composition further contains (1) one or more fillers, such as 1, 2, 3, 4, or 5 fillers; (2) one or more disintegrants, such as 1, 2, 3, 4, or 5 disintegrants; (3) optionally one or more surfactants, such as 1, 2, 3, 4, or 5 surfactants; (4) optionally one or more glidants, such as 1, 2, 3, 4, or 5 glidants; (5) optionally one or more lubricants, such as 1, 2, 3, 4, or 5 lubricants; and (6) optionally one or more film coating premixes, such as 1, 2, 3, 4, or 5 film coating premixes.

Furthermore, the filler is selected from one or more of the following: lactose (such as lactose monohydrate or anhydrous lactose), starch, calcium hydrogen phosphate (such as anhydrous calcium hydrogen phosphate or calcium hydrogen phosphate dihydrate), calcium sulfate dihydrate, mannitol, sorbitol, and microcrystalline cellulose. Furthermore, the microcrystalline cellulose is microcrystalline cellulose PH101. Furthermore, the disintegrant is selected from one or more of the following: croscarmellose sodium, crospovidone (such as crospovidone XL-10, crospovidone CL), pregelatinized starch, and sodium starch glycolate. Furthermore, the surfactant is sodium lauryl sulfate. Furthermore, the glidant is colloidal silicon dioxide. Furthermore, the lubricant is magnesium stearate and/or glyceryl tribehenate.

Furthermore, the filler is present in an amount of 30% to 70% based on a weight of the second pharmaceutical composition. Furthermore, the filler is present in an amount of 55% to 65% such as about 58.5% based on the weight of the second pharmaceutical composition. Furthermore, the disintegrant is present in an amount of 6% to 20% based on the weight of the second pharmaceutical composition. Furthermore, the disintegrant is present in an amount of 10% to 20% such as about 15% based on the weight of the second pharmaceutical composition. Furthermore, the surfactant is present in an amount of 1% to 6% based on the weight of the second pharmaceutical composition. Furthermore, the glidant is present in an amount of 0.5% to 3% such as about 1% based on the weight of the second pharmaceutical composition. Furthermore, the lubricant is present in an amount of 0.5% to 3% such as about 1.5% based on the weight of the second pharmaceutical composition. Furthermore, the film coating premix is present in an amount of 0.5% to 5% such as about 3% based on the weight of the second pharmaceutical composition. Furthermore, the antioxidant is present in an amount of 0.01% to 1% such as about 0.1% based on the weight of the second pharmaceutical composition.

Furthermore, the second pharmaceutical composition is in a form of an oral preparation. Furthermore, the second pharmaceutical composition is in a form of a solid oral preparation, such as a tablet or a capsule.

According to one aspect of the invention, the present invention provides a method for preparing the aforementioned first pharmaceutical composition, wherein the method comprises the following steps: (a) mixing and dissolving the compound of formula (I) or the pharmaceutically acceptable salt thereof with the one or more polymer materials, such as 1, 2, 3, 4, or 5 polymer materials, one or more solvents, such as 1, 2, 3, 4, or 5 solvents, and optionally the one or more antioxidants, such as 1, 2, 3, 4, or 5 antioxidants, to form a mixed solution; (b) spray drying the mixed solution to obtain a mixed powder; and (c) vacuum drying or double cone drying the mixed powder to obtain the first pharmaceutical composition.

Furthermore, the solvent is selected from one or more of the following: water, methanol, acetone, ethanol, isopropanol, acetonitrile, ethyl acetate, propylene glycol, and dichloromethane. Furthermore, the solvent is selected from one or more of the following: water, methanol, ethanol, acetone, and dichloromethane. Furthermore, the solvent is water, methanol, ethanol, acetone, and/or dichloromethane. Furthermore, a mass ratio of the acetone to the methanol is 1:1 to 3:1, such as about 2:1. Furthermore, the solvent is about 70% acetone and methanol, and about 30% water. Furthermore, the solvent is about 40% acetone and methanol, and about 60% dichloromethane.

Furthermore, a mass ratio of the solvent to the first pharmaceutical composition is 85:15 to 97:3. Furthermore, the mass ratio of the solvent to the first pharmaceutical composition is 90:10 to 94:6.

Furthermore, conditions for the spray drying are as follows: setting an inlet air temperature of 70°C to 90°C, an outlet air temperature of 45°C to 55°C, a pump power of 20% to 45%, a condenser temperature of -15°C to -5°C, and an atomizing gas flow rate of 3 kg/h to 12 kg/h. Furthermore, conditions for the vacuum drying are as follows: after a vacuum degree stabilizes at ≤ -0.1 MPa, drying at 55°C to 65°C for 12 h to 48 h. Furthermore, conditions for the double cone drying are as follows: after a vacuum degree stabilizes at ≤ -0.1 MPa, drying at 55°C to 65°C for 4 h to 24 h.

According to one aspect of the invention, the present invention provides a method for preparing the aforementioned first pharmaceutical composition, the method comprises the following steps: (a) sieving and uniformly mixing the compound of formula (I) or the pharmaceutically acceptable salt thereof with the one or more polymer materials, such as 1, 2, 3, 4, or 5 polymer materials, and optionally one or more plasticizers, such as 1, 2, 3, 4, or 5 plasticizers to obtain a mixture; and (b) setting temperatures of segments of a screw of a hot melt extruder, adjusting a feeding speed to 5 rpm to 20 rpm and a screw rotation speed to 20 rpm to 80 rpm to perform hot melt extrusion, optionally cooling and milling, to obtain the first pharmaceutical composition.

Furthermore, the plasticizer is selected from one or more of the following: dibutyl sebacate, diethyl phthalate, Poloxamer F-68, Poloxamer F-127, glyceryl monostearate, polyethylene glycol, sodium lauryl sulfate, dioctyl sodium sulfosuscinate, triethyl citrate, triacetin, Tween 80, and Vitamin E-TPGS.

Furthermore, the temperatures are an initial segment temperature of 20°C to 40°C, a conveying segment temperature of 110°C to 130°C, a mixing segment temperature of 160°C to 180°C, a kneading segment temperature of 160°C to 180°C, and a conveying and discharging segment temperature of 150°C to 170°C.

According to one aspect of the invention, the present invention provides a method for preparing the aforementioned first pharmaceutical composition, the method comprises the following steps: (a) mixing and dissolving the compound of formula (I) or the pharmaceutically acceptable salt thereof with the one or more polymer materials, such as 1, 2, 3, 4, or 5 polymer materials, and one or more solvents, such as 1, 2, 3, 4, or 5 solvents to form a mixed solution; and (b) removing the solvent using a rotary evaporator at 45°C to 65°C, milling the mixture after rotary drying, and sieving through an 18-mesh to 80-mesh sieve to obtain the first pharmaceutical composition.

Furthermore, the solvent is selected from one or more of the following: water, methanol, acetone, ethanol, isopropanol, acetonitrile, ethyl acetate, propylene glycol, and dichloromethane. Furthermore, the solvent is selected from one or more of the following: water, methanol, ethanol, acetone, and dichloromethane. Furthermore, the solvent is water, ethanol, acetone, and/or dichloromethane. Furthermore, a mass ratio of the acetone to the methanol is 1:1 to 3:1, such as about 2:1. Furthermore, the solvent is about 70% acetone and methanol, and about 30% water. Furthermore, the solvent is about 40% acetone and methanol, and about 60% dichloromethane.

Furthermore, a mass ratio of the solvent to the first pharmaceutical composition is 85:15 to 97:3. Furthermore, the mass ratio of the solvent to the first pharmaceutical composition is 90:10 to 94:6.

According to one aspect of the invention, the present invention provides a method for preparing the aforementioned second pharmaceutical composition, the method comprises the following steps: (a) preparing a mixed solution containing the compound of the present invention (i.e., a compound of formula (I), referred to as API) and one or more polymeric materials (optionally, one or more antioxidants such as 1, 2, 3, 4, or 5 antioxidants), spraying the mixed solution onto an appropriate amount of filler and/or disintegrant, drying and sieving to obtain a first pharmaceutical mixture; and (b) mixing the first pharmaceutical mixture with a suitable filler, disintegrant, lubricant, and the like, then further compressing into tablets, filling into capsules, or packing into granules, thereby obtaining tablets, capsules, or granules of the second pharmaceutical composition, respectively.

Furthermore, the method comprises the following steps: (a) mixing and dissolving the compound of formula (I) or the pharmaceutically acceptable salt thereof with the one or more polymer materials, such as 1, 2, 3, 4, or 5 polymer materials, one or more solvents, such as 1, 2, 3, 4, or 5 solvents, and optionally the one or more antioxidants, such as 1, 2, 3, 4, or 5 antioxidants, to form a mixed solution; (b) adding the filler and the disintegrant to a fluidized bed, and preheating; (c) spraying the mixed solution of the first pharmaceutical composition into the fluidized bed, drying, and sieving obtained granules; (d) mixing the sieved granules with an appropriate amount of filler, disintegrant, glidant, and/or lubricant to obtain a second pharmaceutical mixture; and (e) optionally, dispensing and tableting the second pharmaceutical mixture to obtain a tablet of the second pharmaceutical composition; (f) optionally, dispensing and filling capsules with the second pharmaceutical mixture to obtain a capsule of the second pharmaceutical composition; (g) optionally, dispensing and bagging the second pharmaceutical mixture to obtain a granule of the second pharmaceutical composition.

Furthermore, conditions for one-step granulation comprise a fluidized bed inlet air temperature of 45°C to 75°C, a fluidized bed material temperature of 35°C to 55°C, a fluidized bed inlet air volume of 15 m³/h to 80 m³/h, a fluidized bed spray rate of 5 g/min to 50 g/min, and a fluidized bed atomization pressure of 0.05 bar to 1.0 bar.

According to one aspect of the invention, the present invention provides a method for preparing the aforementioned second pharmaceutical composition, the method comprises the following steps: (a) performing a first mixing of the first pharmaceutical composition with an appropriate amount of filler, disintegrant, glidant, and lubricant to obtain a first pharmaceutical mixture; (b) performing dry granulation on the first pharmaceutical mixture to obtain granules; (c) performing a second mixing of the granules with an appropriate amount of disintegrant and lubricant to obtain a second pharmaceutical mixture; and (d) optionally, dispensing and tableting the second pharmaceutical mixture to obtain a tablet of the second pharmaceutical composition; (e) optionally, dispensing and filling capsules with the second pharmaceutical mixture to obtain a capsule of the second pharmaceutical composition; (f) optionally, dispensing and bagging the second pharmaceutical mixture to obtain a granule of the second pharmaceutical composition.

Furthermore, a rotational speed for the first mixing is 10 rpm to 20 rpm, and a mixing time is 10 min to 15 min. Furthermore, a rotational speed for the second mixing is 10 rpm to 20 rpm, and a mixing time is 2 min to 10 min.

Furthermore, in the step (a), the disintegrant is crospovidone and pregelatinized starch. Furthermore, in the step (c), the disintegrant is crospovidone.

Furthermore, a mass ratio of the crospovidone added in the step (a) to the crospovidone added in the step (c) is about 1.

Furthermore, a mass ratio of the lubricant added in the step (a) to the lubricant added in the step (c) is about 2.

Furthermore, conditions for the dry granulation are as follows: a hydraulic pressure of 18 bar to 50 bar, a roller rotation speed of 3.4 rpm to 14.8 rpm, a roller gap of 1 mm to 4 mm, a secondary granulation speed of 24.9 rpm to 108.4 rpm, and a primary granulation speed of 10 rpm to 90 rpm.

Furthermore, a specification for a primary sizing screen in the dry granulation is 2.0 mm, and a specification for a secondary sizing screen in the dry granulation is 0.8 mm.

According to one aspect of the invention, the present invention provides a use of the aforementioned first pharmaceutical composition or the aforementioned second pharmaceutical composition in preparation of a medicament for modulating a protein kinase and/or for treating a protein kinase-mediated disease, disorder, and/or pathology.

Furthermore, the disease, disorder, and/or pathology is a cancer, a vascular disorder, a hypoxic disorder, osteoporosis, or a disease affecting cell motility, adhesion, and cell cycle progression.

Furthermore, the cancer is leukemia, lung cancer, liver cancer, endometrial cancer, gastric cancer, rectal cancer, colon cancer, CNS cancer, melanoma, ovarian cancer, renal cancer, prostate cancer, or breast cancer.

Furthermore, the vascular disorder is myocardial infarction, stroke, or ischemia.

### BRIEF DESCRIPTION OF THE DRAWINGS

In order to more clearly illustrate the technical solutions in the embodiments of the present invention, the drawings used in the description of the embodiments will be briefly introduced below. It is obvious that the drawings in the following description are only some embodiments of the present invention. For those of ordinary skill in the art, other drawings may be obtained based on these drawings without departing from the scope of protection claimed by the present invention.
Figure 1 is a schematic diagram of the dissolution curves of the API+PVP physical mixture and the API+PVP solid dispersions.
Figure 2 is a schematic diagram of the dissolution curves during the tablet experimental stage.
Figure 3 is a schematic diagram of the dissolution curves of the tablet formulations for filler screening.
Figure 4 is a schematic diagram (1) of the dissolution curves of the tablet formulations for screening filler types and addition methods in different dissolution media.
Figure 5 is a schematic diagram (2) of the dissolution curves of the tablet formulations for screening filler types and addition methods in different dissolution media.
Figure 6 is a schematic diagram of the dissolution curves of the tablet formulations for investigating the amount and addition method of the disintegrant P.Starch.
Figure 7 is a schematic diagram of the dissolution curves of the tablet formulations for investigating the amount and addition method of the disintegrant crospovidone.

### DETAILED DESCRIPTION

The technical solutions in the embodiments of the present invention will be clearly and completely described below with reference to the drawings in the embodiments of the present invention. It is apparent that the described embodiments are only a part of the embodiments of the present invention, and not all of the embodiments. All other embodiments obtained by a person of skill in the art based on the embodiments of the present invention without creative efforts shall fall within the scope of protection of the present invention.

The singular forms "a," "an," and "the" as used in the specification and the appended claims include plural referents, unless the context clearly dictates otherwise.

Unless otherwise stated, all technical and scientific terms and abbreviations used herein have the meanings commonly understood by a person of ordinary skill in the art of the present invention or in the field where the term is applied. Although any methods, conditions, substances, or materials similar or equivalent to those disclosed herein may be used in the practice of the present invention, preferred methods, conditions, substances, or materials are described herein.

In the present invention, the term "comprising" is synonymous with "including." The terms "comprising," "including," "having," "containing," or any other variations thereof used herein are intended to cover non-exclusive inclusion. For example, a composition, medicinal material, component, step, method, product, or device that comprises a list of elements is not necessarily limited to only those elements, but may include other elements not explicitly listed or inherent to such composition, medicinal material, component, step, method, product, or device.

The excipients involved in the present invention are merely exemplary. The excipients actually used in the present invention are not limited to those listed herein. The use of excipients can be adjusted according to practical applications, and the effects of the present invention can still be achieved, all of which fall within the scope of protection of the present invention.

As described in the Background Art section, no relevant reports on the pharmaceutical composition containing a substituted indol-5-ol derivative and use thereof as claimed in the present invention have been found in the prior art. To address the above issues, the present invention provides a first pharmaceutical composition containing a substituted indol-5-ol derivative, the first pharmaceutical composition contains
(a) a therapeutically effective amount of a compound of formula (I), or a pharmaceutically acceptable salt thereof; and 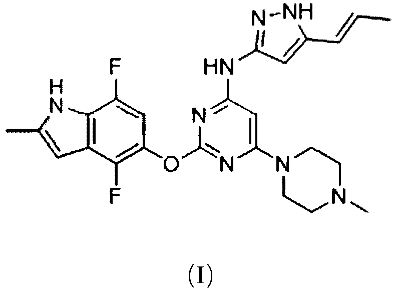
(b) one or more polymer materials, such as 1, 2, 3, 4, or 5 polymer materials.

In a preferred embodiment, the polymer material is selected from one or more of the following: hypromellose acetate succinate, hydroxyalkyl cellulose, povidone, copovidone, polypropylene resin, polyvinyl caprolactam-polyvinyl acetate-polyethylene glycol graft copolymer, and polyethylene glycol. In a preferred embodiment, the hypromellose acetate succinate is hypromellose acetate succinate AS-MMP and/or hypromellose acetate succinate AS-MF. In a preferred embodiment, the hydroxyalkyl cellulose is hydroxypropyl cellulose, hypromellose, and/or hydroxyethyl cellulose. In a preferred embodiment, the hydroxypropyl cellulose is hydroxypropyl cellulose EXF, hydroxypropyl cellulose HXF, and/or hydroxypropyl cellulose LXF. In a preferred embodiment, the hypromellose is hypromellose E3, hypromellose E5, and/or hypromellose E15. In a preferred embodiment, the povidone is povidone K25, povidone K30, povidone K-12, and/or povidone K-17. In a preferred embodiment, the copovidone is copovidone S630. In a preferred embodiment, the polypropylene resin is polypropylene resin E100. In a preferred embodiment, the polyethylene glycol is polyethylene glycol 4000 and/or polyethylene glycol 6000. In a preferred embodiment, the polymer material is povidone K30, hypromellose E3, hypromellose E15, hypromellose acetate succinate AS-MMP, hydroxypropyl cellulose EXF, copovidone S630, polyethylene glycol 4000, polyvinyl caprolactam-polyvinyl acetate-polyethylene glycol graft copolymer, or polypropylene resin E100. In a preferred embodiment, a mass ratio of the compound of formula (I) to the polymer material is 1:1 to 1:10. In a preferred embodiment, the mass ratio of the compound of formula (I) to the polymer material is 1:1 to 1:6. In a preferred embodiment, the mass ratio of the compound of formula (I) to the polymer material is 1:3 to 1:6.

In the present invention, when mass ratios, mass percentages, volume percentages, power, flow rates, time, intensity of pressure, speed, pressure, air volume, rotational speed, gap, concentration, wavelength, temperature, flow rate, parts by weight, or other values or parameters are expressed as a range, a preferred range, or a range defined by a series of upper limit preferred values and lower limit preferred values, this should be understood as specifically disclosing all ranges formed by any pairing of any upper limit or preferred value of the range with any lower limit or preferred value of the range, regardless of whether the range is disclosed separately. For example, when a range such as "1:1 to 1:10" is disclosed, unless otherwise stated, the range is intended to include the endpoints and all integers and fractions within the range, that is, at least including 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, and 1:10.

In a preferred embodiment, the first pharmaceutical composition further contains one or more antioxidants, such as 1, 2, 3, 4, or 5 antioxidants. In a preferred embodiment, the antioxidant is selected from one or more of the following: butylated hydroxyanisole, butylated hydroxytoluene, sodium metabisulfite, vitamin E, vitamin E acetate, and L-ascorbyl palmitate. In a preferred embodiment, the antioxidant is sodium metabisulfite. In a preferred embodiment, the antioxidant is present in an amount of 0.01% to 3% such as about 0.2% based on a weight of the first pharmaceutical composition.

In this invention, "about" refers to a value within ±5% of a specific value. For example, "about 0.2" includes ±5% of 0.2, that is, from 0.19 to 0.21.

In a preferred embodiment, the first pharmaceutical composition is in a form of a solid dispersion.

According to one aspect of the present invention, the present invention provides a second pharmaceutical composition comprising the aforementioned first pharmaceutical composition, wherein the second pharmaceutical composition further contains (1) one or more fillers, such as 1, 2, 3, 4, or 5 fillers; (2) one or more disintegrants, such as 1, 2, 3, 4, or 5 disintegrants; (3) optionally one or more surfactants, such as 1, 2, 3, 4, or 5 surfactants; (4) optionally one or more glidants, such as 1, 2, 3, 4, or 5 glidants; (5) optionally one or more lubricants, such as 1, 2, 3, 4, or 5 lubricants; and (6) optionally one or more film coating premixes, such as 1, 2, 3, 4, or 5 film coating premixes.

In a preferred embodiment, the filler is selected from one or more of the following: lactose (such as lactose monohydrate or anhydrous lactose), starch, calcium hydrogen phosphate (such as anhydrous calcium hydrogen phosphate or calcium hydrogen phosphate dihydrate), calcium sulfate dihydrate, mannitol, sorbitol, and microcrystalline cellulose. In a preferred embodiment, the microcrystalline cellulose is microcrystalline cellulose PH101. In a preferred embodiment, the disintegrant is selected from one or more of the following: croscarmellose sodium, crospovidone (such as crospovidone XL-10, crospovidone CL), pregelatinized starch, and sodium starch glycolate. In a preferred embodiment, the surfactant is sodium lauryl sulfate. In a preferred embodiment, the glidant is colloidal silicon dioxide. In a preferred embodiment, the lubricant is magnesium stearate and/or glyceryl tribehenate.

In a preferred embodiment, the filler is present in an amount of 30% to 70% based on a weight of the second pharmaceutical composition. In a preferred embodiment, the filler is present in an amount of 55% to 65% such as about 58.5% based on the weight of the second pharmaceutical composition. In a preferred embodiment, the disintegrant is present in an amount of 6% to 20% based on the weight of the second pharmaceutical composition. In a preferred embodiment, the disintegrant is present in an amount of 10% to 20% such as about 15% based on the weight of the second pharmaceutical composition. In a preferred embodiment, the surfactant is present in an amount of 1% to 6% based on the weight of the second pharmaceutical composition. In a preferred embodiment, the glidant is present in an amount of 0.5% to 3% such as about 1% based on the weight of the second pharmaceutical composition. In a preferred embodiment, the lubricant is present in an amount of 0.5% to 3% such as about 1.5% based on the weight of the second pharmaceutical composition. In a preferred embodiment, the film coating premix is present in an amount of 0.5% to 5% such as about 3% based on the weight of the second pharmaceutical composition. In a preferred embodiment, the antioxidant is present in an amount of 0.01% to 1% such as about 0.1% based on the weight of the second pharmaceutical composition.

In this invention, "about" refers to a value within ±5% of a specific value. For example, "about 58.5" includes ±5% of 58.5, that is, from 55.575 to 61.425; "about 15" includes ±5% of 15, that is, from 14.25 to 15.75; "about 1" includes ±5% of 1, that is, from 0.95 to 1.05; "about 1.5" includes ±5% of 1.5, that is, from 1.425 to 1.575; "about 3" includes ±5% of 3, that is, from 2.85 to 3.15; "about 0.1" includes ±5% of 0.1, that is, from 0.095 to 0.105.

In a preferred embodiment, the second pharmaceutical composition is in a form of an oral preparation. Furthermore, the second pharmaceutical composition is in a form of a solid oral preparation, such as a tablet.

According to one aspect of the invention, the present invention provides a method for preparing the aforementioned first pharmaceutical composition, the method comprises the following steps: (a) mixing and dissolving the compound of formula (I) or the pharmaceutically acceptable salt thereof with the one or more polymer materials, such as 1, 2, 3, 4, or 5 polymer materials, one or more solvents, such as 1, 2, 3, 4, or 5 solvents, and optionally the one or more antioxidants, such as 1, 2, 3, 4, or 5 antioxidants, to form a mixed solution; (b) spray drying the mixed solution to obtain a mixed powder; and (c) vacuum drying or double cone drying the mixed powder to obtain the first pharmaceutical composition.

In a preferred embodiment, the solvent is selected from one or more of the following: water, methanol, acetone, ethanol, isopropanol, acetonitrile, ethyl acetate, propylene glycol, and dichloromethane. In a preferred embodiment, the solvent is selected from one or more of the following: water, methanol, ethanol, acetone, and dichloromethane. In a preferred embodiment, the solvent is water, methanol, ethanol, acetone, and/or dichloromethane. In a preferred embodiment, a mass ratio of the acetone to the methanol is 1:1 to 3:1, such as about 2:1. In a preferred embodiment, the solvent is about 70% acetone and methanol, and about 30% water. In a preferred embodiment, the solvent is about 40% acetone and methanol, and about 60% dichloromethane.

In the present invention, "about" refers to a value within ±5% of a specific value. For example, "about 2:1" includes ±5% of 2:1, that is, from 1.9:1 to 2.1:1; "about 70" includes ±5% of 70, that is, from 66.5 to 73.5; "about 30" includes ±5% of 30, that is, from 28.5 to 31.5; "about 40" includes ±5% of 40, that is, from 38 to 42; "about 60" includes ±5% of 60, that is, from 57 to 63.

The saturated solubility of the compound of the present invention in different solvents/media is shown in Table 1 (Test method: An appropriate excess amount of API was placed in an appropriate amount of solvent and shaken at room temperature for 24 hours, after which the sample solution was processed and analyzed). Among them, methanol and acetone are the optimal choices.

**Table 1 Saturated Solubility of the Compound of the Present Invention in Different Solvents/Media**

| Solvent or Medium Name | Solubility (mg/mL) | Solubility Description |
|---|---|---|
| Methanol | 8.36 | Slightly soluble |
| Ethanol | 5.17 | Slightly soluble |
| Isopropanol | 2.34 | Slightly soluble |
| Acetone | 5.99 | Slightly soluble |
| Acetonitrile | 1.22 | Slightly soluble |
| Ethyl acetate | 4.98 | Slightly soluble |
| Propylene glycol | 2.22 | Slightly soluble |

The solvent compositions capable of simultaneously dissolving the compound of the present invention and the carrier materials are shown in Table 2. The detection method for the solubility of API in different solvent combinations is as follows: Weigh 20 mg of the compound of the present invention and add it to 4 mL of different solvents or solvent combinations, respectively. Gently shake and observe the dissolution phenomenon. If complete dissolution occurs, add a small amount of additional API to the above solution, continue shaking, and observe the dissolution phenomenon. The results show that the solubility of API in the solvent combinations listed in Table 2 is greater than 5 mg/mL. All carrier materials listed in Table 2 are soluble in the selected solvents or solvent combinations.

**Table 2 Solvent Compositions Capable of Simultaneously Dissolving the Compound of the Present Invention and the Carrier**

| Carrier | Solvent (w/w) | Ratio of Compound of the Present Invention + Carrier Material / Solvent or Solvent Combination |
|---|---|---|
| Hypromellose Acetate Succinate | Acetone: Methanol 2:1 | 3:97~15:85 |
| Hypromellose E15 | 70% (Acetone:Methanol 2:1) + 30% Water | 3:97~10:90 |
| Hypromellose E5 | 40% (Acetone:Methanol 2:1) + 60% Dichloromethane | 3:97~10:90 |
| Povidone K30 | Acetone: Methanol 2:1 | 3:97~15:85 |
| Polypropylene Resin E100 | Acetone: Methanol 2:1 | 3:97~15:85 |
| Copovidone S630 | Acetone: Methanol 2:1 | 3:97~15:85 |
| Soluplus | Acetone: Methanol 2:1 | 3:97~15:85 |
| Povidone K30 | Acetone: Methanol 1:1 | 3:97~12:88 |
| Povidone K30 | Acetone:Methanol 1:2 | 3:97~12:88 |
| Povidone K30 | Acetone:Methanol 4:1 | 3:97~12:88 |
| Povidone K30 | Acetone: Methanol 1:4 | 3:97~12:88 |
| Povidone K30 | Acetone: Ethanol 1:1 | 3:97~12:88 |
| Povidone K30 | Acetone:Ethanol 2:1 | 3:97~12:88 |
| Povidone K30 | Acetone:Ethanol 4:1 | 3:97~12:88 |

In a preferred embodiment, a mass ratio of the solvent to the first pharmaceutical composition is 85:15 to 97:3. In a preferred embodiment, the mass ratio of the solvent to the first pharmaceutical composition is 90:10 to 94:6.

In a preferred embodiment, conditions for the spray drying are as follows: setting an inlet air temperature of 70°C to 90°C, an outlet air temperature of 45°C to 55°C, a pump power of 20% to 45%, a condenser temperature of -15°C to -5°C, and an atomizing gas flow rate of 3 kg/h to 12 kg/h. In a preferred embodiment, conditions for the vacuum drying are as follows: after a vacuum degree stabilizes at ≤ -0.1 MPa, drying at 55°C to 65°C for 12 h to 48 h. In a preferred embodiment, conditions for the double cone drying are as follows: after a vacuum degree stabilizes at ≤ -0.1 MPa, drying at 55°C to 65°C for 4 h to 24 h.

According to one aspect of the invention, the present invention provides a method for preparing the aforementioned first pharmaceutical composition, the method comprises the following steps: (a) sieving and uniformly mixing the compound of formula (I) or the pharmaceutically acceptable salt thereof with the one or more polymer materials, such as 1, 2, 3, 4, or 5 polymer materials, and optionally one or more plasticizers, such as 1, 2, 3, 4, or 5 plasticizers to obtain a mixture; and (b) setting temperatures of segments of a screw of a hot melt extruder, adjusting a feeding speed to 5 rpm to 20 rpm and a screw rotation speed to 20 rpm to 80 rpm to perform hot melt extrusion, optionally cooling and milling, to obtain the first pharmaceutical composition.

The compound of the present invention (API) has a melting point of 195-198°C and is a high-melting-point compound. It is necessary to select a carrier material with an appropriate glass transition temperature for the development of the hot melt extrusion process.

To reduce the temperature required for hot melt extrusion and improve the fluidity in the molten state, in a preferred embodiment, the plasticizer is selected from one or more of the following: dibutyl sebacate, diethyl phthalate, Poloxamer F-68, Poloxamer F-127, glyceryl monostearate, polyethylene glycol, sodium lauryl sulfate, dioctyl sodium sulfosuscinate, triethyl citrate, triacetin, Tween 80, and Vitamin E-TPGS.

In a preferred embodiment, the temperatures are an initial segment temperature of 20°C to 40°C, a conveying segment temperature of 110°C to 130°C, a mixing segment temperature of 160°C to 180°C, a kneading segment temperature of 160°C to 180°C, and a conveying and discharging segment temperature of 150°C to 170°C.

According to one aspect of the invention, the present invention provides a method for preparing the aforementioned first pharmaceutical composition, wherein the method comprises the following steps: (a) mixing and dissolving the compound of formula (I) or the pharmaceutically acceptable salt thereof with the one or more polymer materials, such as 1, 2, 3, 4, or 5 polymer materials, and one or more solvents, such as 1, 2, 3, 4, or 5 solvents to form a mixed solution; and (b) removing the solvent using a rotary evaporator at 45°C to 65°C, milling the mixture after rotary drying, and sieving through an 18-mesh to 80-mesh sieve to obtain the first pharmaceutical composition.

In a preferred embodiment, the solvent is selected from one or more of the following: water, methanol, acetone, ethanol, isopropanol, acetonitrile, ethyl acetate, propylene glycol, and dichloromethane. In a preferred embodiment, the solvent is selected from one or more of the following: water, methanol, ethanol, acetone, and dichloromethane. In a preferred embodiment, the solvent is water, ethanol, acetone, and/or dichloromethane. In a preferred embodiment, a mass ratio of the acetone to the methanol is 1:1 to 3:1, such as about 2:1. In a preferred embodiment, the solvent is about 70% acetone and methanol, and about 30% water. In a preferred embodiment, the solvent is about 40% acetone and methanol, and about 60% dichloromethane.

In a preferred embodiment, a mass ratio of the solvent to the first pharmaceutical composition is 85:15 to 97:3. In a preferred embodiment, the mass ratio of the solvent to the first pharmaceutical composition is 90:10 to 94:6.

According to one aspect of the invention, the present invention provides a method for preparing the aforementioned second pharmaceutical composition, wherein the method comprises the following steps: (a) preparing a mixed solution containing the compound of the present invention (API) and one or more polymeric materials (optionally, one or more antioxidants such as 1, 2, 3, 4, or 5 antioxidants), spraying the mixed solution onto an appropriate amount of filler and/or disintegrant, drying and sieving to obtain a first pharmaceutical mixture; and (b) mixing the first pharmaceutical mixture with a suitable filler, disintegrant, lubricant, and the like, then further compressing into tablets, filling into capsules, or packing into granules, thereby obtaining tablets, capsules, or granules of the second pharmaceutical composition, respectively.

In a preferred embodiment, the method comprises the following steps: (a) mixing and dissolving the compound of formula (I) or the pharmaceutically acceptable salt thereof with the one or more polymer materials, such as 1, 2, 3, 4, or 5 polymer materials, one or more solvents, such as 1, 2, 3, 4, or 5 solvents, and optionally the one or more antioxidants, such as 1, 2, 3, 4, or 5 antioxidants, to form a mixed solution; (b) adding the filler and the disintegrant to a fluidized bed, and preheating; (c) spraying the mixed solution of the first pharmaceutical composition into the fluidized bed, drying for 10 to 30 min, and sieving obtained granules; (d) mixing the sieved granules with an appropriate amount of filler, disintegrant, glidant, and/or lubricant to obtain a second pharmaceutical mixture; and (e) optionally, dispensing and tableting the second pharmaceutical mixture to obtain a tablet of the second pharmaceutical composition; (f) optionally, dispensing and filling capsules with the second pharmaceutical mixture to obtain a capsule of the second pharmaceutical composition; (g) optionally, dispensing and bagging the second pharmaceutical mixture to obtain a granule of the second pharmaceutical composition.

In a preferred embodiment, conditions for one-step granulation comprise a fluidized bed inlet air temperature of 45°C to 75°C, a fluidized bed material temperature of 35°C to 55°C, a fluidized bed inlet air volume of 15 m³/h to 80 m³/h, a fluidized bed spray rate of 5 g/min to 50 g/min, and a fluidized bed atomization pressure of 0.05 bar to 1.0 bar.

According to one aspect of the invention, the present invention provides a method for preparing the aforementioned second pharmaceutical composition, the method comprises the following steps: (a) performing a first mixing of the first pharmaceutical composition with an appropriate amount of filler, disintegrant, glidant, and lubricant to obtain a first pharmaceutical mixture; (b) performing dry granulation on the first pharmaceutical mixture to obtain granules; (c) performing a second mixing of the granules with an appropriate amount of disintegrant and lubricant to obtain a second pharmaceutical mixture; and (d) optionally, dispensing and tableting the second pharmaceutical mixture to obtain a tablet of the second pharmaceutical composition; (e) optionally, dispensing and filling capsules with the second pharmaceutical mixture to obtain a capsule of the second pharmaceutical composition; (f) optionally, dispensing and bagging the second pharmaceutical mixture to obtain a granule of the second pharmaceutical composition.

In a preferred embodiment, a rotational speed for the first mixing is 10 rpm to 20 rpm, and a mixing time is 10 min to 15 min. In a preferred embodiment, a rotational speed for the second mixing is 10 rpm to 20 rpm, and a mixing time is 2 min to 10 min.

In a preferred embodiment, in the step (a), the disintegrant is crospovidone and pregelatinized starch. In a preferred embodiment, in the step (c), the disintegrant is crospovidone.

In a preferred embodiment, a mass ratio of the crospovidone added in the step (a) to the crospovidone added in the step (c) is about 1.

In a preferred embodiment, a mass ratio of the lubricant added in the step (a) to the lubricant added in the step (c) is about 2.

In a preferred embodiment, conditions for the dry granulation are as follows: a hydraulic pressure of 18 bar to 50 bar, a roller rotation speed of 3.4 rpm to 14.8 rpm, a roller gap of 1 mm to 4 mm, a secondary granulation speed of 24.9 rpm to 108.4 rpm, and a primary granulation speed of 10 rpm to 90 rpm.

In a preferred embodiment, a specification for a primary sizing screen in the dry granulation is 2.0 mm, and a specification for a secondary sizing screen in the dry granulation is 0.8 mm.

According to one aspect of the invention, the present invention provides a use of the aforementioned first pharmaceutical composition or the aforementioned second pharmaceutical composition in preparation of a medicament for modulating a protein kinase and/or for treating a protein kinase-mediated disease, disorder, and/or pathology.

In a preferred embodiment, the disease, disorder, and/or pathology is a cancer, a vascular disorder, a hypoxic disorder, osteoporosis, or a disease affecting cell motility, adhesion, and cell cycle progression.

In a preferred embodiment, the cancer is leukemia, lung cancer, liver cancer, endometrial cancer, gastric cancer, rectal cancer, colon cancer, CNS cancer, melanoma, ovarian cancer, renal cancer, prostate cancer, or breast cancer.

In a preferred embodiment, the vascular disorder is myocardial infarction, stroke, or ischemia.

According to one aspect of the present invention, the present invention provides the aforementioned first pharmaceutical composition or the aforementioned second pharmaceutical composition for modulating a protein kinase and/or treating a protein kinase-mediated disease, disorder, and/or pathology.

According to one aspect of the present invention, the present invention provides a method for modulating a protein kinase and/or treating a protein kinase-mediated disease, disorder, and/or pathology, comprising administering an effective amount of the aforementioned first pharmaceutical composition or the aforementioned second pharmaceutical composition to a subject.

In the present invention, the term "subject" refers to a mammal. The mammal may be a human, a non-human primate, a mouse, a rat, a dog, a cat, a horse, or a cow, but is not limited to these examples. Mammals other than humans may be advantageously used as subjects representing models for modulating a protein kinase and/or treating a protein kinase-mediated disease, disorder, and/or pathology. Preferably, the subject is a human.

An "effective amount" of the pharmaceutical composition or formulation used in the present invention can achieve the desired therapeutic and/or prophylactic effect. The amount effective for this use will depend on factors such as the pharmaceutical composition, the administration route, the stage and severity of the disease being treated, the individual's body weight and overall health, and the judgment of the prescribing physician. The administration of the dose may be once per week, or once every two days, or once daily, or even several times per day. The dosage unit may be administered over a short period (for example, weeks to months) or over a longer period (months to years).

The present invention is further described below with reference to specific examples. It should be understood that these examples are only for illustrating the present invention and not for limiting the scope of the present invention. Experimental methods for which specific conditions are not indicated in the following examples are generally carried out under conventional conditions or under conditions recommended by the manufacturer.

Unless otherwise defined, all professional and scientific terms used herein have the same meanings as those familiar to a person skilled in the art. Furthermore, any methods and materials similar or equivalent to those described herein may be applied in the methods of the present invention. The preferred embodiments and materials described herein are for illustrative purposes only.

The aforementioned features mentioned in the present invention, or the features mentioned in the examples, may be combined arbitrarily. All features disclosed in this patent specification may be used in combination with any compound form. Each feature disclosed in the specification may be replaced by any alternative feature that serves the same, equivalent, or similar purpose. Therefore, unless specifically stated otherwise, the disclosed features are merely general examples of equivalent or similar features.

### Example

### 1. Examples of Preparing Solid Dispersion Using Povidone

### 1.1 Formulation Composition

**Table 3 Summary of Formulations for Examples of Preparing Solid Dispersion Using Povidone**

| | Ratio (w/w) % | | | | | | | |
|---|---|---|---|---|---|---|---|---|
| Material Name | Example 1 | Example 2 | Example 3 | Example 4 | Example 5 | Example 6 | Example 7 | Example 8 |
| Compound of the Invention (API ) | 66.7 | 50.0 | 33.3 | 25.0 | 20.0 | 16.7 | 14.3 | 9.09 |
| Povi done K30 | / | 50.0 | 66.7 | 75.0 | 80.0 | 83.3 | 85.7 | 90.91 |
| Povidone K90 | 33.3 | / | / | / | / | / | / | / |
| Solvent Used | / | / | Acetone: Methanol =2:1 | Acetone: Methanol =2:1 | Acetone: Methanol =2:1 | Acetone: Methanol =2:1 | Acetone: Methanol =2:1 | Acetone: Methanol =2:1 |
| | Acetone: Ethanol-4:1 | Acetone: Ethanol-4:1 | / | / | / | / | / | / |
| Solid Content | 6% | 6% | 6% | 8% | 10% | 6% | 6% | 10% |

| | | | | | | | | |
|---|---|---|---|---|---|---|---|---|
| Note: The batch size for the examples listed in Table 3 is 10 g per batch, and the solvent amount is calculated based on the solid content. | | | | | | | | |

### 1.2 Preparation Method

### 1.2.1 Preparation Method for Example 1

Weigh the API and Povidone K90 into a rotary evaporation flask, add an appropriate amount of acetone:ethanol = 4:1 (w/w) mixed solvent to ensure complete dissolution of the API and PVP K90, and remove the solvent using a rotary evaporator at 55°C. Take the dried mixture out of the rotary evaporation flask, mill it, and sieve it through a 24-mesh sieve to obtain the product.

### 1.2.2 Preparation Method for Example 2

Weigh the API and Povidone K30 into a rotary evaporation flask, add an appropriate amount of acetone:ethanol = 4:1 (w/w) mixed solvent to ensure complete dissolution of the API and PVP K30, and remove the solvent using a rotary evaporator at 55°C. Take the dried mixture out of the rotary evaporation flask, mill it, and sieve it through a 40-mesh sieve to obtain the product.

### 1.2.3 Preparation Method for Examples 3-6

1) Material Preparation: Weigh the API, excipients, and solvent according to the formula amounts.
2) Drug Solution Preparation: Prepare a mixed solvent of acetone:methanol = 2:1, add the API to this mixed solvent, and stir magnetically until the solution becomes clear. Then add PVP K30 and continue stirring until dissolved to obtain a clear solution.
3) Spray Drying:
   a. Start and set the fan power to 80% to 90%, and the nitrogen flow valve to 30 mm to 40 mm, to achieve system balance.
   b. After the system is balanced, set the inlet air temperature to 85°C (70°C to 85°C) for preheating. Once the measured temperature reaches the set temperature, spray the acetone-methanol mixed solvent, adjust the pump power to 20% to 45%, and balance the system until the outlet air temperature reaches 45°C to 55°C.
   c. Spray the drug solution to prepare the solid dispersion.
4) Vacuum Drying: Collect the sample, transfer it to a vacuum drying oven, and dry at 60°C under vacuum for 24 h to 48 h.

### 1.2.4 Preparation Method for Examples 7-8

Weigh the API and PVP K30 into a rotary evaporation flask, add an appropriate amount of acetone:methanol = 2:1 (w/w) mixed solvent to ensure complete dissolution of the API and PVP K30, and remove the solvent using a rotary evaporator at 55°C. Take the dried mixture out of the rotary evaporation flask, mill it, and sieve it through a 24-mesh sieve to obtain the product.

### 1.3 Solubility Testing

Weigh an amount of powder (equivalent to 50 mg of API) from Examples 1-7 into a 10-mL vial, add 5 mL of media with different pH values, so that the theoretical concentration of API in the sample solution was about 10 mg/mL. Shake the vials in a shaker for 24 hours, and determine the solubility. During shaking, observe the dissolution phenomenon of the powder in the solution. If the solution becomes clear, add an additional amount of sample powder. The results are shown in Table 4.

**Table 4 Solubility of Solid Dispersions Prepared Using Povidone in Different Media**

| Example | | Example 1 | Example 2 | Example 6 | Example 7 | Example 3 | Example 4 | Example 5 | Compound of the Invention (API) |
|---|---|---|---|---|---|---|---|---|---|
| Theoretical API Concentration in Sample | | about 10 mg/mL | | | | Supersaturated | | | Supersaturated |
| Medium | Time | Solubility (mg/mL) | | | | | | | |
| 0.1mol/ L HCl | 4h | 1.2 | 2.6 | 8.9 | 9.3 | 11.0 | 9.6 | 8.9 | 0.05 |
| | 24h | 0.8 | 1.1 | 9.0 | 9.2 | 3.0 | 7.9 | 7.9 | |
| pH6.8 | 4h | 0.07 | 1.2 | 9.1 | 9.6 | 0.1 | 1.2 | 2.6 | 0.00(Below Detection Limit) |
| | 24h | 0.05 | 1.0 | 9.2 | 9.9 | 0.4 | 1.3 | 3.2 | |

The results show that for solid dispersions prepared using povidone, when the drug-excipient ratio is between 1:0.5 and 1:10, the solubility of the API can be enhanced. The preferred drug-excipient ratio is 1:1 to 1:6, and a more preferred ratio is 1:3 to 1:6.

### 1.4 Comparison of Dissolution Curves Between API+PVP Physical Mixture and API+PVP Solid Dispersions

Preparation method of API:PVP K30 = 1:6 physical mixture: Weigh one part of the compound of the present invention and six parts of povidone K30, mix uniformly to obtain the physical mixture of the two.

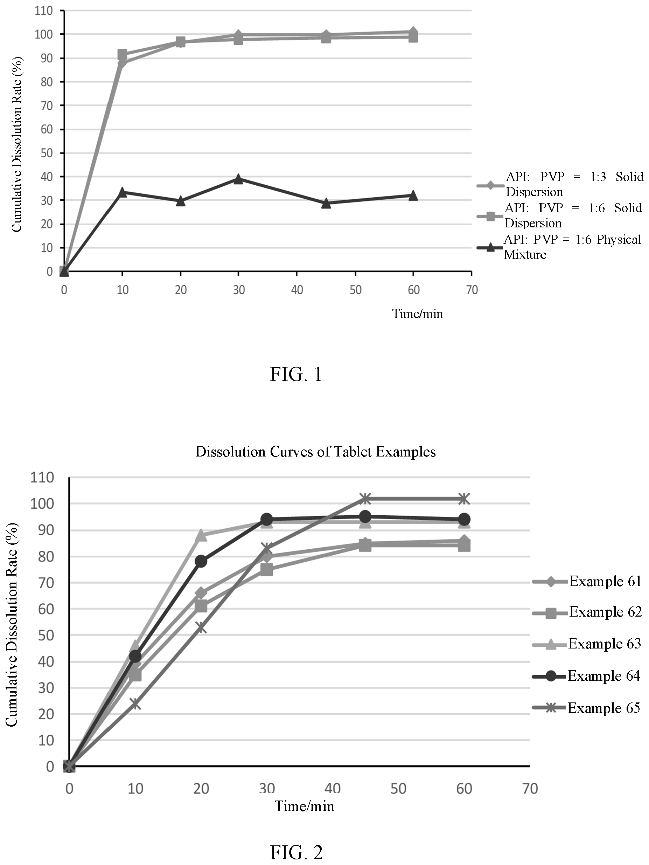

The dissolution method was as follows: Paddle method, 75 RPM, 37°C ± 0.5°C, 900 mL of 0.1 mol/L HCl medium. The dissolution results are shown in Table 5 and Figure 1.

**Table 5 Dissolution Results**

| Cumulative Dissolution Rate (%) | Time / min | | | | | |
|---|---|---|---|---|---|---|
| | 0 | 10 | 20 | 30 | 45 | 60 |
| Example 4 Solid Dispersion | 0 | 88.0 | 96.5 | 99.7 | 99.9 | 101.1 |
| Example 7 Solid Dispersion | 0 | 91.6 | 97.0 | 97.7 | 98.4 | 98.7 |
| API:PVP=1:6 Physical Mixture | 0 | 33.5 | 29.8 | 39.2 | 28.9 | 32.3 |

The results show that in 0.1 mol/L HCl medium, the physical mixture of API and povidone K30 does not dissolve completely, whereas the solid dispersions achieve complete dissolution within a short time.

### 1.5 XRPD Patterns

The XRPD patterns indicate that Examples 2-8 all formed amorphous substances.

### 2. Examples of Preparing Solid Dispersions Using Hypromellose

### 2.1 Formulation Composition

**Table 6 Summary of Formulations for Examples of Preparing Solid Dispersions Using Hypromellose**

| Material Name | Ratio (w/w) % | | | | |
|---|---|---|---|---|---|
| | Example 9 | Example 10 | Example 11 | Example 12 | Example 13 |
| Compound of the | 50 | 25 | 20 | 14.3 | 25 |
| Invention (API) | | | | | |
| HPMC E5 | 50 | 75 | 80 | 85.7 | / |
| HPMC E15 | / | / | / | / | 75 |
| Solvent Used | (Acetone:M ethanol=2:1 ): Dichloromethane=4:6 | (Acetone:Meth anol=2:1): Dichloromethane=4 :6 | (Acetone:Met hanol=2:1): Dichloromethane=4:6 | (Acetone: Methanol =2:1): Dichloromethane=4:6 | / |
| | / | / | / | / | (Acetone: Methanol =2:1): Purified Water=7: 3 |
| Solid Content | 4% | 4% | 4% | 4% | 5.8% |

| | | | | | |
|---|---|---|---|---|---|
| Note: The batch size for the examples listed in Table 6 is 10 g per batch, and the solvent amount is calculated based on the solid content. | | | | | |

### 2.2 Preparation Method

### 2.2.1 Preparation Method for Examples 9-12

Weigh the API and HPMC E5 into a rotary evaporation flask, add an appropriate amount of acetone:methanol = 2:1 (w/w) + dichloromethane mixed solvent (ratio 4:6 (w/w)) to ensure complete dissolution of the API and HPMC E5, and remove the solvent using a rotary evaporator at 55°C. Take the dried mixture out of the rotary evaporation flask, mill it, and sieve it through a 24-mesh sieve for later use.

### 2.2.2 Preparation Method for Example 13

Dissolution: Weigh the API and excipients according to the formula amounts, add hypromellose E15 to the (acetone:methanol 2:1):water = 7:3 mixed solvent, stir to dissolve until the solution was clear, then add the API, and stir until the API was completely dissolved to obtain a yellowish clear solution.

Spray Drying: Subject the drug solution to spray drying by setting the fan frequency to 75%-85%, nitrogen flow rate to 590-710 L/h, inlet air temperature to 90-125°C, and spray rate to 3-5 g/min.

Drying: After spray drying was completed, collect the sample and dry it in a vacuum drying oven at 60°C for 24 hours (until the residual solvent meets the standard), and then take out the material.

### 2.3 Solubility Testing

Weigh an amount of powder (equivalent to 50 mg of API) from Examples 9-13 into a 10-mL vial, add a corresponding volume of media with different pH values, so that the theoretical concentration of API in the solution was about 10 mg/mL (the theoretical concentration of API in the sample solution of Example 13 was about 5 mg/mL). Shake the vials in a shaker for 24 hours, and sample at 4 h and 24 h to determine the solubility. During shaking, observe the dissolution phenomenon of the powder in the solution. If the solution becomes clear, add an additional amount of sample powder. The results are shown in Table 7.

**Table 7 Solubility of Solid Dispersions Prepared Using HPMC in Different Media**

| Example | | Exampl e 9 | Exampl e 10 | Exampl e 11 | Exampl e 12 | Exampl e 13 | API |
|---|---|---|---|---|---|---|---|
| Sample Concentration | | about 10 mg/mL | | | | about 5 mg/mL | |
| Medium | Tim e | Solubility (mg/mL) | | | | | |
| 0.1mol/ L HCl | 4h | 0.4 | 2.2 | 8.2 | 7.8 | 3.3 | 0.05 |
| | 24h | 0.2 | 0.7 | 8.1 | 8.5 | 3.6 | |
| pH6.8 | 4h | 0.04 | 0.06 | 0.1 | 0.19 | 0.03 | 0.00(Belo w Detection Limit) |
| | 24h | 0.03 | 0.08 | 0.1 | 0.2 | 0.06 | |

The results show that for solid dispersions prepared using HPMC E5 and HPMC E15, when the drug-excipient ratio is between 1:1 and 1:6, the solubility of the API can be enhanced. Preferably, when the drug-excipient ratio is between 1:4 and 1:6, the solubilization effect is optimal.

### 2.4 XRPD Patterns

XRPD patterns indicate that Examples 9-13 all formed amorphous substances.

### 3. Examples of Preparing Solid Dispersions Using Hydroxypropyl Cellulose

### 3.1 Formulation Composition

HPC EXF was selected as the carrier material for the preparation of solid dispersions, as detailed in Table 8.

**Table 8 Summary of Formulations for Examples of Preparing Solid Dispersions Using Hydroxypropyl Cellulose**

| Material Name | Ratio (w/w) % | | |
|---|---|---|---|
| | Example 14 | Example 15 | Example 16 |
| Compoun d of the Invention (API) | 50.0 | 16.7 | 14.3 |
| HPC EXF | 50.0 | 83.3 | 85.7 |
| Solvent Used | Acetone: Methanol=2: 1 | Acetone:Methanol=2: 1 | Acetone:Methanol=2: 1 |
| Solid Content | 6% | 6% | 6% |

| | | | |
|---|---|---|---|
| Note: The batch size for the examples listed in Table 8 is 10 g per batch, and the solvent amount is calculated based on the solid content. | | | |

### 3.2 Preparation Process

Weigh the API and HPC EXF into a rotary evaporation flask, add an appropriate amount of acetone:methanol = 2:1 (w/w) mixed solvent, and stir to completely dissolve the API and HPC EXF. Remove the solvent using a rotary evaporator at 45-55°C. Take the dried mixture out of the rotary evaporation flask, mill it, and sieve it through a 24-mesh sieve.

### 3.3 Solubility Testing

Weigh an amount of powder (equivalent to 50 mg of API) from Examples 14-16 into a 10-mL vial, add a corresponding volume of media with different pH values, so that the theoretical concentration of API in the sample solution was about 10 mg/mL. Shake the vials in a shaker for 24 hours, and sample at 4 h and 24 h to determine the solubility. During shaking, observe the dissolution phenomenon of the powder in the solution. If the solution becomes clear, add an additional amount of sample powder. The results are shown in Table 9.

**Table 9 Solubility of Solid Dispersions Prepared Using HPC in Different Media**

| Example | | Example 14 | Example 15 | Example 16 | API |
|---|---|---|---|---|---|
| Sample Concentration | | about 10 mg/mL | | | |
| Medium | Time | Solubility (mg/mL) | | | |
| 0.1mol/L HCl | 4h | 0.25 | 2.63 | 2.78 | 0.05 |
| | 24h | 0.23 | 5.06 | 5.67 | |
| pH6.8 | 4h | 0.08 | 0.13 | 0.09 | 0.00 (Below Detection Limit) |
| | 24h | 0.11 | 0.09 | 0.1 | |

The results show that for solid dispersions prepared using HPC EXF, when the drug-excipient ratio is between 1:1 and 1:6, the solubility of the API can be enhanced.

### 3.4 XRPD Patterns

XRPD patterns indicate that Examples 14-16 all formed amorphous substances.

### 4. Examples of Preparing Solid Dispersions Using Copovidone

### 4.1 Formulation Composition

**Table 10 Summary of Formulations for Examples of Preparing Solid Dispersions Using Copovidone S630**

| Material Name | Ratio (w/w) % | | | | |
|---|---|---|---|---|---|
| | Example 17 | Example 18 | Example 19 | Example 20 | Example 21 |
| Compoun d of the Invention (API) | 50.0 | 33.3 | 25.0 | 20.0 | 14.3 |
| Copovido ne S630 | 50.0 | 66.7 | 75.0 | 80.0 | 85.7 |
| Solvent Used | / | / | Acetone:Me thanol=2:1 | Acetone:Me thanol=2:1 | Acetone:M ethanol=2: 1 |
| | Acetone | Acetone | / | / | / |
| Solid Content | 6% | 6% | 6% | 6% | 6% |

| | | | | | |
|---|---|---|---|---|---|
| Note: The batch size for the examples listed in Table 10 is 10 g per batch, and the solvent amount is calculated based on the solid content. | | | | | |

### 4.2 Preparation Process

### 4.2.1 Preparation Method for Examples 17-18

Weigh the API and Copovidone S630 into a rotary evaporation flask, add an appropriate amount of acetone to ensure complete dissolution of the API and Copovidone S630, and remove the solvent using a rotary evaporator at 45°C to 55°C. Take the dried mixture out of the rotary evaporation flask, mill it, and sieve it through a 24-mesh sieve.

### 4.2.2 Preparation Method for Example 19

1) Material Preparation: Weigh the API, excipients, and solvent according to the formula amounts.
2) Drug Solution Preparation: Add the API and Copovidone S630 to a mixed solvent of acetone:methanol = 2:1, and stir until completely dissolved to obtain a clear solution.
3) Spray Drying:
   a. Start and set the fan power to 80% to 90%, and the nitrogen flow valve to 30 mm to 40 mm, to achieve system balance.
   b. After the system is balanced, set the inlet air temperature to 85°C (70°C to 85°C) for preheating. Once the measured temperature reaches the set temperature, spray the acetone-methanol mixed solvent, adjust the pump power to 20% to 45%, and balance the system until the outlet air temperature reaches 45°C to 55°C.
   c. Spray the drug solution to prepare the solid dispersion.
4) Vacuum Drying: Collect the sample, transfer it to a vacuum drying oven, and dry at 60°C under vacuum for 24 h to 48 h.

### 4.2.3 Preparation Method for Examples 20-21

Weigh the API and Copovidone S630 into a rotary evaporation flask, add an appropriate amount of acetone:methanol = 2:1 (w/w) mixed solvent to ensure complete dissolution of the API and Copovidone S630, and remove the solvent using a rotary evaporator at 55°C. Take the dried mixture out of the rotary evaporation flask, mill it, and sieve it through a 24-mesh sieve.

### 4.3 Solubility Testing

Weigh an amount of powder (equivalent to 50 mg of API) from Examples 17-21 into a 10-mL vial, add a corresponding volume of media with different pH values, so that the theoretical concentration of API in the sample solution was about 10 mg/mL. Shake the vials in a shaker for 24 hours, and sample at 4 h and 24 h to determine the solubility. During shaking, observe the dissolution phenomenon of the powder in the solution. If the solution becomes clear, add an additional amount of sample powder. The results are shown in Table 11.

**Table 11 Solubility of Solid Dispersions Prepared Using Copovidone S630 in Different Media**

| Example | | Exampl e 17 | Exampl e 18 | Exampl e 20 | Exampl e 21 | Exampl e 19 | API |
|---|---|---|---|---|---|---|---|
| Sample Concentration | | about 10 mg/mL | | | | about 5 mg/mL | |
| Medium | Tim e | Solubility (mg/mL) | | | | | |
| 0.1mol/ L HCl | 4h | 1.5 | 9.0 | 9.1 | 8.4 | >5 | 0.05 |
| | 24h | 1.0 | 8.8 | 8.9 | 9.1 | >5 | |
| pH6.8 | 4h | 0.4 | 0.3 | 1.1 | 4.2 | 0.2 | 0 (Below Detectio n Limit) |
| | 24h | 0.5 | 0.9 | 1.4 | 4.7 | 0.3 | |

The results show that for solid dispersions prepared using Copovidone S630, when the drug-excipient ratio is between 1:1 and 1:6, the solubility of the API can be enhanced. Preferably, the drug-excipient ratio is 1:3 to 1:6.

### 4.4 XRPD Patterns

The XRPD patterns indicate that Examples 17-21 all formed amorphous substances.

### 5. Examples of Preparing Solid Dispersions Using HPMC AS

### 5.1 Formulation Composition

**Table 12 Summary of Formulations for Examples of Preparing Solid Dispersions Using HPMC AS**

| Material Name | Ratio (w/w) % | | |
|---|---|---|---|
| | Example 22 | Example 23 | Example 24 |
| Compoun d of the Invention (API) | 50.0 | 25.0 | 14.3 |
| HPMC AS | 50.0 | 75.0 | 85.7 |
| Solvent Used | Acetone: Methanol=2: 1 | Acetone:Methanol=2: 1 | Acetone:Methanol=2: 1 |
| Solid Content | 5% | 8% | 5% |

| | | | |
|---|---|---|---|
| Note: The batch size for the examples listed in Table 12 is 10 g per batch, and the solvent amount is calculated based on the solid content. | | | |

### 5.2 Preparation Process

### 5.2.1 Preparation Method for Example 22 and Example 24

Weigh the API and HPMC AS into a rotary evaporation flask, add an appropriate amount of acetone:methanol = 2:1 (w/w) mixed solvent to ensure complete dissolution of the API and HPMC AS, and remove the solvent using a rotary evaporator at 55°C. Take the dried mixture out of the rotary evaporation flask, mill it, and sieve it through a 24-mesh sieve.

### 5.2.2 Preparation Method for Example 23

1) Material Preparation: Weigh the API, excipients, and solvent according to the formula amounts.
2) Drug Solution Preparation: Prepare a mixed solvent of acetone:methanol = 2:1, add the API to this mixed solvent, and stir magnetically until the solution becomes clear. Then add HPMC AS and continue stirring until dissolved to obtain a clear solution.
3) Spray Drying:
   a. Start and set the fan power to 80% to 90%, and the nitrogen flow valve to 30 mm to 40 mm, to achieve system balance.
   b. After the system is balanced, set the inlet air temperature to 85°C (70°C to 85°C) for preheating. Once the measured temperature reaches the set temperature, spray the acetone-methanol mixed solvent, adjust the pump power to 20% to 45%, and balance the system until the outlet air temperature reaches 45°C to 55°C.
   c. Spray the drug solution to prepare the solid dispersion.
4) Vacuum Drying: Collect the sample, transfer it to a vacuum drying oven, and dry at 60°C under vacuum for 24 h to 48 h.

### 5.3 Solubility Determination

Weigh an amount of powder (equivalent to 50 mg of API) from Examples 22-24 into a 10-mL vial, add a corresponding volume of media with different pH values, so that the theoretical concentration of API in the sample solution was about 10 mg/mL. Shake the vials in a shaker for 24 hours, and sample at 4 h and 24 h to determine solubility. The results are shown in Table 13.

**Table 13 Solubility of Solid Dispersions Prepared Using HPMC AS in Different Media**

| Example | | Example 22 | Example 23 | Example 24 | API |
|---|---|---|---|---|---|
| Sample Concentration | | about 10 mg/mL | | about 5 mg/mL | |
| Medium | Time | Solubility (mg/mL) | | | |
| 0.1mol/L HCl | 4h | 4.60 | 0.24 | 1.79 | 0.05 |
| | 24h | 2.21 | 0.92 | 0.62 | |
| pH6.8 | 4h | 0.01 | 0.02 | 0.02 | 0 (Below Detection Limit) |
| | 24h | 0.01 | 0.02 | 0.04 | |

The results show that for solid dispersions prepared using HPMC AS, when the drug-excipient ratio is between 1:1 and 1:6, the solubility of the API can be enhanced.

### 5.4 XRPD Patterns

The XRPD patterns indicate that Examples 22-24 all formed amorphous substances.

### 6. Preparation of Solid Dispersions Using Polyethylene Glycol (Solid, Molecular Weight Greater Than 1000)

### 6.1 Formulation Composition

**Table 14 Summary of Formulations for Examples of Preparing Solid Dispersions Using Polyethylene Glycol**

| Material Name | Ratio (w/w) % | | | |
|---|---|---|---|---|
| | Example 25 | Example 26 | Example 27 | Example 28 |
| Compound of the Invention (API) | 50.0 | 20.0 | 14.3 | 20.0 |
| PEG 4000 | 50.0 | 80.0 | 85.7 | / |
| PEG 6000 | / | / | / | 80.0 |
| Solvent Used | Acetone:Methanol=2:1 | Acetone:Methanol=2:1 | Acetone:Methanol=2:1 | Acetone:Methanol=2:1 |
| Solid Content | 10% | 10% | 10% | 10% |

| | | | | |
|---|---|---|---|---|
| Note: The batch size for the examples listed in Table 14 is 10 g per batch, and the solvent amount is calculated based on the solid content. | | | | |

### 6.2 Preparation Process

Weigh the API and carrier material into a rotary evaporation flask, add an appropriate amount of acetone:methanol = 2:1 (w/w) mixed solvent to ensure complete dissolution of the API and carrier material, and remove the solvent using a rotary evaporator at 45°C. Take the dried mixture out of the rotary evaporation flask, mill it, and sieve it through a 24-mesh sieve for later use.

### 6.3 Solubility Determination

Weigh an amount of powder(equivalent to 50 mg of API) from Examples 25-28 into a 10 mL vial, add a corresponding volume of media with different pH values, so that the theoretical concentration of API in the sample solution was about 10 mg/mL. Shake the vials in a shaker for 24 hours, and sample at 4 h and 24 h to determine the solubility. During shaking, observe the dissolution phenomenon of the powder in the solution. If the solution becomes clear, add an additional amount of sample powder. The results are shown in Table 15.

**Table 15 Solubility of Solid Dispersions Prepared Using Polyethylene Glycol in Different Media**

| Example | | Example 25 | Example 26 | Example 27 | Example 29 | API |
|---|---|---|---|---|---|---|
| Sample Concentration | | about 10 mg/mL | | | | |
| Medium | Time | Solubility (mg/mL) | | | | |
| 0.1mol/L HCl | 4h | 0.18 | 0.3 | 0.46 | 0.38 | 0.05 |
| | 24h | 0.17 | 0.25 | 0.44 | 0.36 | |
| pH6.8 | 4h | 0.02 | 0.05 | 0.07 | 0.06 | 0 (Below Detection Limit) |
| | 24h | 0.02 | 0.03 | 0.05 | 0.05 | |

The results show that for solid dispersions prepared using polyethylene glycol, when the drug-excipient ratio is between 1:1 and 1:6, the prepared solid dispersions can all enhance solubility.

### 6.4 XRPD Patterns

The XRPD patterns indicate that Examples 25-28 all formed amorphous substances.

### 7. Preparation of Solid Dispersions Using Polypropylene Resin E100 (Eudragit^{®} E100)

### 7.1 Formulation Composition

**Table 16 Summary of Formulations for Solid Dispersions Prepared Using Eudragit^{®} E100**

| Material Name | Ratio (w/w) % | |
|---|---|---|
| | Example 29 | Example 30 |
| Compound of the Invention (API) | 25.0 | 10.0 |
| Eudragit^{®} E100 | 75.0 | 90.0 |
| Solvent Used | Acetone:Methanol=2:1 | Acetone: Methanol=2:1 |
| Solid Content | 8% | 5% |

| | | |
|---|---|---|
| Note: The batch size for the examples listed in Table 16 is 10 g per batch, and the solvent amount is calculated based on the solid content. | | |

### 7.2 Preparation Process

1) Material Preparation: Weigh the API, excipients, and solvent according to the formula amounts.
2) Drug Solution Preparation: Prepare a mixed solvent of acetone:methanol = 2:1, add the API to this mixed solvent, and stir magnetically until the solution becomes clear. Then add Eudragit^{®} E100 and continue stirring until dissolved to obtain a clear solution.
3) Spray Drying:
   a. Start and set the fan power to 80% to 90%, and the nitrogen flow valve to 30 mm to 40 mm, to achieve system balance.
   b. After the system is balanced, set the inlet air temperature to 85°C (70°C to 85°C) for preheating. Once the measured temperature reaches the set temperature, spray the acetone-methanol mixed solvent, adjust the pump power to 20% to 45%, and balance the system until the outlet air temperature reaches 45°C to 55°C.
   c. Spray the drug solution to prepare the solid dispersion.
4) Vacuum Drying: Collect the sample, transfer it to a vacuum drying oven, and dry at 60°C under vacuum for 24 h.

### 7.3 Solubility Testing

Weigh an amount of powder (equivalent to 50 mg of API) from Examples 29-30 into a 10-mL vial, add a corresponding volume of media with different pH values, so that the theoretical concentration of API in the sample solution was about 10 mg/mL. Shake the vials in a shaker for 24 hours, and sample at 4 h and 24 h to determine the solubility. During shaking, observe the dissolution phenomenon of the powder in the solution. If the solution becomes clear, add an additional amount of sample powder. The results are shown in Table 17.

**Table 17 Solubility of Solid Dispersions Prepared Using Eudragit^{®} E100 in Different Media**

| Example | | Example 29 | Example 30 | API |
|---|---|---|---|---|
| Sample Concentration | | about 5 mg/mL | about 10 mg/mL | |
| Medium | Time | Solubility (mg/mL) | | |
| 0.1mol/L HCl | 2h | 4.83 | 9.12 | 0.05 |
| | 24h | 1.23 | 8.36 | |
| pH6.8 | 2h | 0.01 | 0.08 | |
| | 24h | 0.01 | 0.12 | 0.00 (Below Detection Limit) |

The results show that the solid dispersions prepared in Example 29 and Example 30 can enhance the solubility of the API.

### 7.4 XRPD Patterns

The XRPD patterns indicate that Examples 29-30 formed amorphous substances.

### 8. Examples of Preparing Solid Dispersions Using Polyvinyl Caprolactam-Polyvinyl Acetate-Polyethylene Glycol Graft Copolymer (Soluplus)

### 8.1 Formulation Composition

**Table 18 Summary of Formulations for Solid Dispersions Prepared Using Soluplus**

| Material Name | Ratio (w/w) % | | |
|---|---|---|---|
| | Example 31 | Example 32 | Example 33 |
| Compound of the Invention (API) | 33.3 | 20 | 14.3 |
| Soluplus | 66.7 | 80 | 85.7 |
| Solvent Used | Acetone: Methanol=2: 1 | Acetone: Methanol=2: 1 | Acetone:Methanol=2: 1 |
| Solid Content | 10% | 10% | 10% |

| | | | |
|---|---|---|---|
| Note: The batch size for the examples listed in Table 18 is 10 g per batch, and the solvent amount is calculated based on the solid content. | | | |

### 8.2 Preparation Process

### 8.2.1 Preparation Method for Example 31 and Example 33

Weigh the API and Soluplus into a rotary evaporation flask, add an appropriate amount of acetone:methanol = 2:1 (w/w) mixed solvent to ensure complete dissolution of the API and Soluplus, and remove the solvent using a rotary evaporator at 45°C to 55°C. Take the dried mixture out of the rotary evaporation flask, disperse it, and sieve it through a 30-mesh sieve for later use.

### 8.2.2 Preparation Method for Example 32

1) Material Preparation: Weigh the API, excipients, and solvent according to the prescribed amounts.
2) Drug Solution Preparation: Prepare a mixed solvent of acetone:methanol = 2:1, add the API to this mixed solvent, and stir magnetically until the solution becomes clear. Then add Soluplus and continue stirring until dissolved to obtain a clear solution.
3) Spray Drying:
   a. Start and set the fan power to 80% to 90%, and the nitrogen flow valve to 30 mm to 40 mm, to achieve system balance.
   b. After system balance, set the inlet air temperature to 85°C (70°C to 85°C) for preheating. Once the measured temperature reaches the set temperature, spray the acetone-methanol mixed solvent, adjust the pump power to 20% to 45%, and balance the system until the outlet air temperature reaches 45°C to 55°C.
   c. Spray the drug solution to prepare the solid dispersion.
4) Vacuum Drying: Collect the sample, transfer it to a vacuum drying oven, and dry at 60°C under vacuum for 24 h to 48 h.

### 8.3 Solubility Testing

Weigh an amount of powder (equivalent to 50 mg of API) from Examples 31-33 into a 10 mL vial, add a corresponding volume of media with different pH values, so that the theoretical concentration of API in the sample solution was about 10 mg/mL. Shake the vials in a shaker for 24 hours, and sample at 4 h and 24 h to determine the solubility. During shaking, observe the dissolution phenomenon of the powder in the solution. If the solution becomes clear, add an additional amount of sample powder. The results are shown in Table 19.

**Table 19 Solubility of Solid Dispersions Prepared Using Soluplus in Different Media**

| Example | | Example 31 | Example 33 | Example 32 | API |
|---|---|---|---|---|---|
| Sample Concentration | | about 10 mg/mL | | Supersaturated | |
| Medium | Time | Solubility (mg/mL) | | | |
| 0.1mol/L HCl | 4h | 0.24 | 0.58 | 0.36 | 0.05 |
| | 24h | 0.33 | 0.65 | 0.52 | |
| pH6.8 | 4h | 0.01 | 0.06 | 0.02 | 0 (Below Detection Limit) |
| | 24h | 0.02 | 0.06 | 0.02 | |

The results show that for solid dispersions prepared using Soluplus, when the drug-excipient ratio is between 1:2 and 1:6, the solubility of the API can be enhanced.

### 8.4 XRPD Patterns

The XRPD patterns indicate that Examples 31-33 all formed amorphous substances.

### 9. Hot Melt Extrusion Formulations and Process

The batch size for the examples of solid dispersions prepared using the hot melt extrusion process was 20 g per batch.

### 9.1 Examples of Preparing Solid Dispersions Using Povidone by Hot Melt Extrusion

### 9.1.1 Formulation Composition

**Table 20 Summary of Formulations**

| Material Name | Ratio (w/w) % | | | | |
|---|---|---|---|---|---|
| | Example 34 | Example 35 | Example 36 | Example 37 | Example 38 |
| Compound of the Invention (API) | 50.0 | 25.0 | 9.0 | 20.0 | 14.3 |
| PVP K-12 | 50.0 | 75.0 | 91.0 | / | / |
| PVP K-17 | / | / | / | 80.0 | 85.7 |

### 9.1.2 Preparation Process

Sieve the API and excipients through a 40-mesh sieve twice and mix thoroughly in a ziplock bag. Set the temperatures of each segment of the screw of the hot melt extruder (as shown in Table 21), adjust the feeding speed to 5-20 rpm, and the screw rotation speed to 20-80 rpm to perform hot melt extrusion. After extrusion, cool the material, and then mill it. The milled material can be directly filled into capsules or mixed with other excipients for tableting.

**Table 21 Hot Melt Extruder Process Parameters**

| Hot Melt Extruder Screw Temperature | Example 34 | Example 35 | Example 36 | Example 37 | Example 38 |
|---|---|---|---|---|---|
| Initial Segment Temperature (°C) | 30 | 30 | 30 | 30 | 30 |
| Conveying Segment Temperature (°C) | 120 | 120 | 120 | 120 | 120 |
| Mixing Segment Temperature (°C) | 170 | 165 | 165 | 165 | 160 |
| Kneading Segment Temperature (°C) | 170 | 165 | 165 | 165 | 160 |
| Conveying and Discharging Segment Temperature (°C) | 155 | 155 | 155 | 155 | 150 |

### 9.1.33 Test Results

The results show that when using povidone as the carrier material (specifically PVP K-12 and PVP K-17), and with drug-excipient ratios within the range of 1:1 to 1:10, amorphous solid dispersions can be prepared by hot melt extrusion.

According to XRPD results, when the drug-excipient ratio is between 1:1 and 1:6, the API and the carrier material povidone (PVP K-12 and PVP K-17) both form amorphous substances.

### 9.2 Examples of Preparing Solid Dispersions Using Copovidone by Hot Melt Extrusion

### 9.2.1 Formulation Composition

**Table 22 Summary of Formulations**

| Material Name | Ratio (w/w) % | | | | | |
|---|---|---|---|---|---|---|
| | Example 39 | Example 40 | Example 41 | Example 42 | Example 43 | Example 44 |
| Compound of the Invention (API) | 45 | 30 | 25 | 20 | 14.3 | 9.1 |
| Copovidone S630 | 45 | 60 | 75 | 80 | 85.7 | 90.9 |
| Triethyl Citrate | 10 | 10 | / | / | / | / |

### 9.2.2 Preparation Process:

Thoroughly mix the API and excipients. Set the temperatures of each segment of the screw of the hot melt extruder (as shown in Table 23), adjust the feeding speed to 5-20 rpm, and the screw rotation speed to 20-80 rpm to perform hot melt extrusion. After extrusion, cool the material, and then mill it. The milled material can be directly filled into capsules or mixed with other excipients for tableting.

**Table 23 Hot Melt Extruder Process Parameters**

| Hot Melt Extruder Screw Temperature | Example 39 | Example 40 | Example 41 | Example 42 | Example 43 | Example 44 |
|---|---|---|---|---|---|---|
| Initial Segment Temperature (°C) | 30 | 30 | 30 | 30 | 30 | 30 |
| Conveying Segment Temperature (°C) | 120 | 120 | 120 | 120 | 120 | 120 |
| Mixing Segment Temperature (°C) | 160 | 160 | 160 | 170 | 170 | 170 |
| Kneading Segment Temperature (°C) | 160 | 160 | 160 | 170 | 170 | 170 |
| Conveying and Discharging Segment Temperature (°C) | 150 | 150 | 150 | 160 | 160 | 160 |

### 9.2.3 Test Results

Using Copovidone S630, amorphous solid dispersions can be prepared by hot melt extrusion within the drug-excipient ratio range of 1:1 to 1:10. If necessary, a certain proportion of plasticizer such as triethyl citrate can be added for hot melt extrusion.

### 9.3 Examples of Preparing Solid Dispersions Using Hypromellose by Hot Melt Extrusion

### 9.3.1 Formulation Composition

**Table 24 Summary of Formulations for HPMC-Based Carrier Materials**

| | Ratio (w/w) % | | | | | |
|---|---|---|---|---|---|---|
| Material Name | Example 45 | Example 46 | Example 47 | Example 48 | Example 49 | Example 50 |
| Compound of the Invention (API) | 50.0 | 25.0 | 14.3 | 9.1 | 25.0 | 20.0 |
| HPMC E3 | 50.0 | 75.0 | 85.7 | 90.9 | / | / |
| HPMC E5 | / | / | / | / | 75.0 | 80.0 |

### 9.3.2 Preparation Process

Thoroughly mix the API and excipients. Set the temperatures of each segment of the screw of the hot melt extruder (as shown in Table 25), adjust the feeding speed to 5-20 rpm, and the screw rotation speed to 20-80 rpm to perform hot melt extrusion. After extrusion, cool the material, and then mill it. The milled material can be directly filled into capsules or mixed with other excipients for tableting.

**Table 25 Hot Melt Extruder Process Parameters**

| Hot Melt Extruder Screw Temperature | Example 45 | Example 46 | Example 47 | Example 48 | Example 49 | Example 50 |
|---|---|---|---|---|---|---|
| Initial Segment Temperature (°C) | 30 | 30 | 30 | 30 | 30 | 30 |
| Conveying Segment Temperature (°C) | 120 | 120 | 120 | 120 | 120 | 120 |
| Mixing Segment Temperature (°C) | 165 | 165 | 165 | 170 | 170 | 170 |
| Kneading Segment Temperature (°C) | 165 | 165 | 165 | 170 | 170 | 170 |
| Conveying and Discharging Segment Temperature (°C) | 155 | 155 | 155 | 160 | 160 | 160 |

### 9.3.3 Test Results

Using HPMC E3 and HPMC E5, amorphous solid dispersions can be prepared by hot melt extrusion within the drug-excipient ratio range of 1:1 to 1:10.

### 9.4 Examples of Preparing Solid Dispersions Using Hydroxypropyl Cellulose by Hot Melt Extrusion

### 9.4.1 Formulation Composition

**Table 26 Summary of Formulations**

| Material Name | Ratio (w/w) % | | | |
|---|---|---|---|---|
| | Example 51 | Example 52 | Example 53 | Example 54 |
| Compound of the Invention (API) | 50.0 | 33.3 | 20.0 | 14.3 |
| HPC EXF | 50.0 | 66.7 | 80.0 | 85.7 |

### 9.4.2 Preparation Process

Sieve the API and excipients through a 40-mesh sieve twice and mix thoroughly in a ziplock bag. Set the temperatures of each segment of the screw of the hot melt extruder (as shown in Table 27), adjust the feeding speed to 5-20 rpm, and the screw rotation speed to 20-80 rpm to perform hot melt extrusion. After extrusion, cool the material, and then mill it. The milled material can be directly filled into capsules or mixed with other excipients for tableting.

**Table 27 Hot Melt Extruder Process Parameters**

| Hot Melt Extruder Screw Temperature | Example 51 | Example 52 | Example 53 | Example 54 |
|---|---|---|---|---|
| Initial Segment Temperature (°C) | 30 | 30 | 30 | 30 |
| Conveying Segment Temperature (°C) | 120 | 120 | 120 | 120 |
| Mixing Segment Temperature (°C) | 170 | 170 | 170 | 170 |
| Kneading Segment Temperature (°C) | 170 | 170 | 170 | 170 |
| Conveying and Discharging Segment Temperature (°C) | 160 | 160 | 160 | 160 |

### 9.4.3 Test Results

XRPD results show that when the ratio of API to HPC EXF is 1:1, amorphous substance is not completely formed; extrudates prepared with drug-excipient ratios of 1:2 to 1:6 are amorphous substances.

### 9.5 Examples of Preparing Solid Dispersions Using Hypromellose Acetate Succinate as Carrier Material by Hot Melt Extrusion

### 9.5.1 Formulation Composition

**Table 28 Summary of Formulations**

| Material Name | Ratio (w/w) % | | | |
|---|---|---|---|---|
| | Example 55 | Example 56 | Example 57 | Example 58 |
| Compound of the Invention (API) | 50.0 | 33.3 | 20.0 | 14.3 |
| HPMC AS-MMP | 50.0 | 66.7 | 80.0 | 85.7 |

### 9.5.2 Preparation Process

Thoroughly mix the API and excipients. Set the temperatures of each segment of the screw of the hot melt extruder (as shown in Table 29), adjust the feeding speed to 5-20 rpm, and the screw rotation speed to 20-80 rpm to perform hot melt extrusion. After extrusion, cool the material, and then mill it. The milled material can be directly filled into capsules or mixed with other excipients for tableting.

**Table 29 Hot Melt Extruder Process Parameters**

| Hot Melt Extruder Screw Temperature | Example 55 | Example 56 | Example 57 | Example 58 |
|---|---|---|---|---|
| Initial Segment Temperature (°C) | 30 | 30 | 30 | 30 |
| Conveying Segment Temperature (°C) | 120 | 120 | 120 | 120 |
| Mixing Segment Temperature (°C) | 170 | 170 | 170 | 170 |
| Kneading Segment Temperature (°C) | 170 | 170 | 170 | 170 |
| Conveying and Discharging Segment Temperature (°C) | 160 | 160 | 160 | 160 |

### 9.5.3 Test Results

Using HPMC AS, amorphous solid dispersions can be prepared by hot melt extrusion within the drug-excipient ratio range of 1:1 to 1:6, or even broader ranges such as 1:1 to 1:10.

### 10. Examples of the Second Pharmaceutical Composition

The first composition obtained by spray drying is typically a loose powder with significant electrostatic charge and poor flowability. It is generally necessary to add one or more excipients such as appropriate fillers, disintegrants, lubricants, and glidants to improve the flowability of the solid dispersion powder, enhance the dissolution rate, and formulate it into oral dosage forms such as tablets, capsules, or granules. Commonly used excipients for oral solid preparations (including but not limited to) are listed in Table 30.

**Table 30 Types of Commonly Used Tablet Excipients**

| Serial Number | Excipient Name | Excipient Function |
|---|---|---|
| 1 | Lactose (monohydrate or anhydrous) | Filler |
| 2 | Starch | Filler |
| 3 | Calcium Hydrogen Phosphate (anhydrous or dihydrate) | Filler |
| 4 | Calcium Sulfate Dihydrate | Filler |
| 5 | MCC PH101 | Filler |
| 6 | Croscarmellose Sodium (CC-Na) | Disintegrant |
| 7 | Crospovidone (PVPP XL-10) | Disintegrant |
| 8 | Crospovidone (PVPP CL) | Disintegrant |
| 9 | Pregelatinized Starch (P.Starch) | Disintegrant |
| 10 | Sodium Starch Glycolate (CMS-Na) | Disintegrant |
| 11 | Sodium Lauryl Sulfate (SDS) | Surfactant |
| 12 | Colloidal Silicon Dioxide (SiO₂) | Glidant and Anti-adherent |
| 13 | Magnesium Stearate (Mg.S) | Lubricant and Anti-adherent |
| 14 | Compritol 888 | Lubricant |

Specific examples are as follows:
10.1 Tablet Examples
10.1.1 Tablet Examples 59 and 60

The formulation compositions are shown in Table 31.

**Table 31 Summary of Example Formulations**

| Material Name | | | Example 59 | | Example 60 | |
|---|---|---|---|---|---|---|
| | | | Formulation Composition % | mg/ tablet | Formulation Composition % | mg/ tablet |
| Internal | Solid Dispersions | API | 12.5 | 22.5 | 10.0 | 22.5 |
| | | Povidone K30 | 37.5 | 67.5 | 40.0 | 90.0 |
| | Lactose | | 25.0 | 45.0 | 25.0 | 56.3 |
| | Microcrystalline Cellulose | | 12.0 | 21.6 | 12.0 | 27.0 |
| | Pregelatinized Starch | | 10.0 | 18.0 | 10.0 | 22.5 |
| | Colloidal Silicon Dioxide | | 2.0 | 3.6 | 2.0 | 4.5 |
| External | Magnesium Stearate | | 1.0 | 1.8 | 1.0 | 2.3 |
| The total core weight | | | 100.0 | 180.0 | 100.0 | 225.0 |

### Preparation Process for Example 59 and Example 60

### (1) Preparation of Solid Dispersion

Considering the yield of the solid dispersion prepared by spray drying, the batch size for the solid dispersion was 50 g per batch, and the required amount for tablets was weighed accordingly.

Material Preparation: Weigh povidone, sodium metabisulfite, and API sequentially according to the batch quantity.

Drug Solution Preparation: Prepare a mixed solvent of acetone:methanol = 2:1. Add the weighed API to the mixing tank and stir until the solution becomes clear. Add the weighed povidone to the above solution and stir until the solution becomes clear, and prepare a drug solution with a solid content of 10% for later use.

Spray Drying: Set the inlet air temperature to 85°C, outlet air temperature to 50°C, and atomizing gas flow rate to 5.5 kg/h (3.0 kg/h to 12.0 kg/h). Spray the drug solution to prepare the solid dispersion.

Vacuum Drying: Set the vacuum drying oven temperature to 60°C, and vacuum degree to ≤ -0.1 MPa, and dry for 12 to 48 hours (until residual solvent testing meets standards).

### (2) Tablet Preparation

Material Preparation: Weigh each material sequentially according to the batch quantity.

Pre-blending: Add the solid dispersion and internal excipients (lactose, microcrystalline cellulose, pregelatinized starch, and colloidal silicon dioxide (internal)) into a mortar, and grind to mix uniformly.

Dry Granulation: (Large Tablet Method) Use a single-punch tablet press to prepare blank tablets. Crush the blank tablets and sieve them through a 24-mesh sieve for later use.

Final Blending: Calculate and weigh magnesium stearate (external) based on the weight of the granules after dry granulation. Thoroughly mix the granules after dry granulation and magnesium stearate (external) in a PE bag.

Tableting: Select Φ 8 mm round punches and use a single-punch tablet press to compress into tablets.

### 10.1.2 Examples of Tablets with Different Drug Loadings

The formulation compositions are shown in Table 32.

**Table 32 Summary of 30 mg Tablet Formulations**

| Material Name | | | Formulation Proportion/% | | | | |
|---|---|---|---|---|---|---|---|
| | | | Example 61 | Example 62 | Example 63 | Example 64 | Example 65 |
| Tablet weight of 30 mg specification core tablet/mg | | | 240 | 240 | 300 | 500 | 500 |
| Internal | Solid Dispersions | API | 12.5 | 12.5 | 10.0 | 6.0 | 6.0 |
| | | PVP K30 | 37.5 | 37.5 | 30.0 | 18.0 | 17.904 |
| | | Sodium Metabisulfite | - | - | - | - | 0.096 |
| | Lactose | | 25.0 | 25.0 | 32.0 | 39.0 | 39.0 |
| | Microcrystalline Cellulose | | 12.0 | 13.0 | 15.5 | 19.5 | 19.5 |
| | Pregelatinized Starch | | 10.0 | 10.0 | 10.0 | 10.0 | 10.0 |
| | Crospovidone | | - | - | - | 2.5 | 2.5 |
| | Colloidal Silicon Dioxide | | 1.0 | 1.0 | 1.0 | 1.0 | 1.0 |
| | Magnesium Stearate | | 0.5 | 0.5 | 1.0 | 1.0 | 1.0 |
| External | Crospovidone | | - | - | - | 2.5 | 2.5 |
| | Colloidal Silicon Dioxide | | 1.0 | - | - | - | - |
| | Magnesium Stearate | | 0.5 | 0.5 | 0.5 | 0.5 | 0.5 |
| Film Coating Premixes | | | 3.0 | 3.0 | 3.0 | 3.0 | 3.0 |

### Preparation Process:

### (1) Preparation of Solid Dispersion:

Material Preparation: Weigh povidone, sodium metabisulfite, and API sequentially according to the batch quantity.

Drug Solution Preparation: Prepare a mixed solvent of acetone/methanol (w/w, 2:1). Add the weighed API to the mixing tank and stir until the solution becomes clear. (For Example 65: Dissolve the weighed sodium metabisulfite in 3 times the amount of purified water, then add it to the mixing tank.) Add the weighed povidone to the mixing tank and stir until the solution becomes clear, and prepare a drug solution with a solid content of 10% for later use.

Spray Drying: Set the inlet air temperature to 85°C (80-90°C), outlet air temperature to 50°C (45-55°C), and atomizing gas flow rate to 6.0 kg/h (3.0 kg/h-12.0 kg/h). Spray the drug solution to prepare the API solid dispersion.

Vacuum Drying: Place the API solid dispersion in a vacuum drying oven, set the temperature to 60°C, and vacuum degree to ≤ -0.1 MPa, and dry for 24-48 hours.

### (2) Tableting

Material Preparation: Weigh each material sequentially according to the batch quantity.

Pre-blending: Add the API solid dispersion, lactose, microcrystalline cellulose, pregelatinized starch, crospovidone (internal), colloidal silicon dioxide, and magnesium stearate (internal) to the blender bin. Set the blending speed to 15 rpm and blend for 12 minutes, then collect the material.

Dry Granulation: Primary sizing screen is 2.0 mm, and the secondary sizing screen is 0.8 mm. Set the roller unit to 6.0 (3.4-14.8) rpm, roller gap to 1.5 (1.0-4.0) mm, hydraulic pressure to 19 (18-50) bar, and secondary granulator to 70.0 (24.9-108.4) rpm. Start the equipment for granulation.

Final Blending: Add the granules after dry granulation and crospovidone (external) to the blender bin. Set the blending speed to 15 rpm and blend for 5 minutes. After blending, add magnesium stearate (external), set the blending speed to 15 rpm, and mix for 2 minutes.

Tableting: Examples 61-62: Use 8 mm round tablet tooling for compression; Example 63: Use 9 mm round tablet tooling for compression; Examples 64-65: Use 15.8*8.2 mm tablet tooling and a rotary tablet press for compression.

Coating: Prepare a coating solution with 12% concentration. After preheating the coating pan and core tablets (add blank tablets if necessary), set the main machine speed to 12.0 (6.0-20.0) rpm, inlet air temperature to 60°C (55-65°C), inlet air volume to 1000 (500-1500) rpm, exhaust air volume to 1200 (800-2900) rpm, peristaltic pump spray rate to 30 (10-100) ml/min, and atomization pressure to 0.2 (0.1-0.5) MPa. Start spraying, control the bed temperature at 37.0-45.0°C, and maintain the coating weight gain at about 3% (2-4%). Dry and cool.

The dissolution results for each batch in experiments are shown in Table 33, and the dissolution curves are shown in Figure 2.

**Table 33 Dissolution Results at the Tablet Experimental Stage**

| Time/min | Dissolution/% | | | | |
|---|---|---|---|---|---|
| | Example 61 | Example 62 | Example 63 | Example 64 | Example 65 |
| 0 | 0 | 0 | 0 | 0 | 0 |
| 10 | 39 | 35 | 46 | 42 | 24 |
| 20 | 66 | 61 | 88 | 78 | 53 |
| 30 | 80 | 75 | 93 | 94 | 83 |
| 45 | 85 | 84 | 93 | 95 | 102 |
| 60 | 86 | 84 | 93 | 94 | 102 |

For Examples 61-62, the dissolution method was: paddle method at 75 rpm, 0.1 mol/L HCl + 0.1% SDS; for Examples 63-65, the dissolution method was: paddle method at 75 rpm, 0.1 mol/L HCl + 0.1% Tween 80.

### 10.1.3 Examples for Tablet Filler Screening

### 10.1.3.1 Examples for Filler Amount Screening

In this section, the tablet examples are based on a drug content of 6% in the tablet formulation and a strength of 30 mg. However, these examples are not limiting in any way and do not represent the final drug content or strength of the formulation. They are provided solely for illustrative purposes.

### 10.1.3.1.1 Formulation Composition

**Table 34 Summary of Tablet Formulations for Filler Amount Screening**

| Material Name | | | Formulation Proportion/% | | | | | | | | |
|---|---|---|---|---|---|---|---|---|---|---|---|
| | | | Tablets Formulation for Screening | | | | | | | | |
| | | | Exa mpl e 64 | Exa mpl e 66 | Exa mpl e 67 | Exa mpl e 68 | Exa mpl e 69 | Exa mpl e 70 | Exa mpl e 71 | Exa mpl e 72 | Exa mpl e 73 |
| Tablet weight of 30 mg strength core tablet/mg | | | 500 | 500 | 500 | 500 | 500 | 500 | 500 | 500 | 500 |
| I n t e r n a l | API Solid | AP I | 6.0 | 6.0 | 6.0 | 6.0 | 6.0 | 6.0 | 6.0 | 6.0 | 6.0 |
| | Dispersi ons | PV P K3 0 | 18.0 | 18.0 | 18.0 | 18.0 | 18.0 | 18.0 | 18.0 | 18.0 | 18.0 |
| | Lactose | | 39.0 19.5 | 58.5 | 46.8 | 43.8 8 | 29.2 5 | 19.5 | 14.6 3 | 11.7 | - |
| | | | | | | | | | | | - |
| | Microcrystalli ne Cellulose | | | - | 11.7 | 14.6 3 | 29.2 5 | 39 | 43.8 8 | 46.8 | 58.5 |
| | | | | - | | | | | | | |
| | Pregelatinized Starch | | 10.0 | 10.0 | 10.0 | 10.0 | 10.0 | 10.0 | 10.0 | 10.0 | 10.0 |
| | Crospovidone | | 2.5 | 2.5 | 2.5 | 2.5 | 2.5 | 2.5 | 2.5 | 2.5 | 2.5 |
| | Colloidal Silicon Dioxide | | 1.0 | 1.0 | 1.0 | 1.0 | 1.0 | 1.0 | 1.0 | 1.0 | 1.0 |
| | Magnesium Stearate | | 1.0 | 1.0 | 1.0 | 1.0 | 1.0 | 1.0 | 1.0 | 1.0 | 1.0 |
| E x t e r n a l | Crospovidone | | 2.5 | 2.5 | 2.5 | 2.5 | 2.5 | 2.5 | 2.5 | 2.5 | 2.5 |
| | Magnesium Stearate | | 0.5 | 0.5 | 0.5 | 0.5 | 0.5 | 0.5 | 0.5 | 0.5 | 0.5 |
| Film Coating Premixes | | | 3.0 | - | - | - | - | - | - | - | - |
| | | | | - | - | - | - | - | - | - | - |
| Note (Lactose/MCC Ratio) | | | 2:1 | 100 %La ctos e | 4:1 | 3:1 | 1:1 | 1:2 | 1:3 | 1:4 | 100 %M CC |

### 10.1.3.1.2 Preparation Process

### (1) Preparation of Solid Dispersion

Material Preparation: Weigh povidone, sodium metabisulfite, and API sequentially according to the batch quantity.

Drug Solution Preparation: Prepare a mixed solvent of acetone:methanol = 2:1. Add the weighed API to the mixing tank and stir until the solution becomes clear. Add the weighed povidone to the above solution and stir until the solution becomes clear, and prepare a drug solution with a solid content of 10% for later use.

Spray Drying: Set the inlet air temperature to 85°C, outlet air temperature to 50°C, and atomizing gas flow rate to 5.5 kg/h (3.0 kg/h to 12.0 kg/h). Spray the drug solution to prepare the solid dispersion.

Vacuum Drying: Set the vacuum drying oven temperature to 60°C, and vacuum degree to ≤ -0.1 MPa, and dry for 12 to 48 hours (until residual solvent testing meets the standards).

### (2) Tablet Preparation

Material Preparation: Weigh each material sequentially according to the batch quantity.

Pre-blending: Add the solid dispersion and internal excipients to the blender bin. Set the blending speed to 15 rpm and blend for 8 minutes, and then collect the material.

Dry Granulation: Set the hydraulic pressure to 20 bar (18 to 230 bar), roller unit speed to 8 rpm (3.4 to 14.8 rpm), roller gap to 1.5 mm, primary sizing speed to 40 rpm, and secondary sizing speed to 50 rpm (24.9 to 108.4 rpm) for granulation.

Final Blending: Calculate and weigh the external excipients based on the weight of the granules after dry granulation. Blend the granules after dry granulation and the external excipients (excluding magnesium stearate) using a bin blender. Set the blending speed to 15 rpm and blend for 5 minutes. Add magnesium stearate (external), set the blending speed to 15 rpm, and blend for 2 minutes.

Tableting: Select 15.8*8.2 mm tablet tooling and use a single-punch tablet press to compress into tablets.

The tablet preparation processes for Examples 66-73 are all feasible.

### 10.1.3.1.3 Test Results

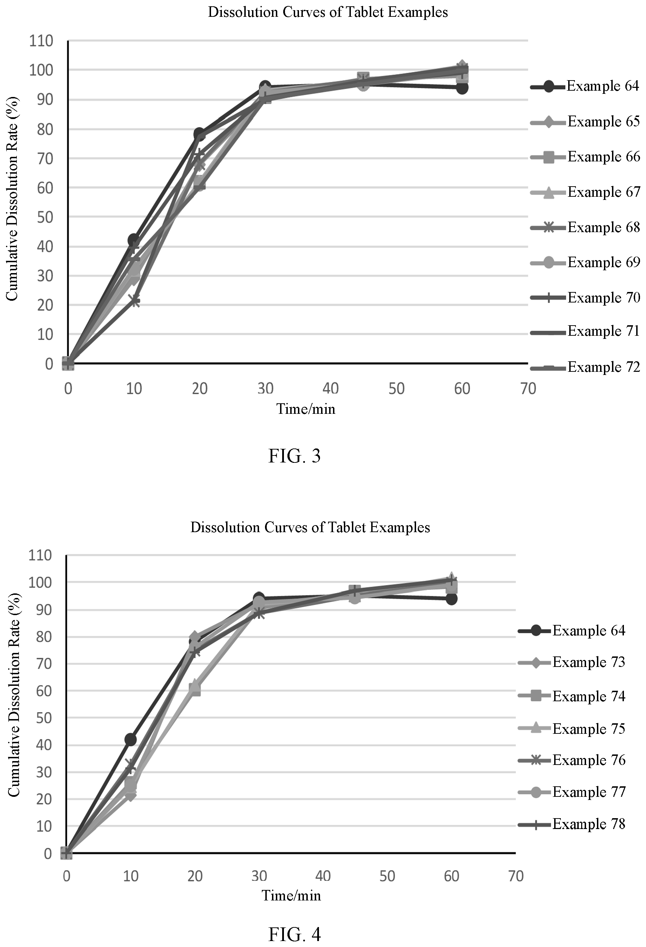

The dissolution data for Examples 64-73 are shown in Table 35 and Figure 3.

**Table 35 Summary of Dissolution Data for Tablet Formulations for Filler Screening**

| Time/ min | Dissolution/% | | | | | | | | |
|---|---|---|---|---|---|---|---|---|---|
| | Exam ple 64 | Exam ple 65 | Exam ple 66 | Exam ple 67 | Exam ple 68 | Exam ple 69 | Exam ple 70 | Exam ple 71 | Exam ple 72 |
| | 30 mg Strength | | | | | | | | |
| 0 | 0 | 0 | 0 | 0 | 0 | 0 | 0 | 0 | 0.0 |
| 10 | 42 | 28.8 | 31 | 32.2 | 21.6 | 32.3 | 39.4 | 21.5 | 35.6 |
| 20 | 78 | 67.7 | 61.7 | 61.8 | 68.4 | 61 | 71.5 | 77.5 | 60.1 |
| 30 | 94 | 91 | 90.8 | 92.9 | 92.2 | 92.4 | 90.6 | 90 | 90.3 |
| 45 | 95 | 95.7 | 97 | 96.1 | 96.6 | 95.2 | 95.8 | 96.1 | 95.1 |
| 60 | 94 | 101.2 | 98.1 | 99.1 | 100.3 | 99.2 | 98.9 | 100.9 | 99.6 |

The dissolution method for the above examples was: paddle method at 75 rpm, 0.1 mol/L HCl + 0.1% Tween 80.

### 10.1.3.2 Examples for Filler Type Screening

### 10.1.3.2.1 Formulation Composition

Mannitol, anhydrous calcium hydrogen phosphate, etc., were selected as fillers for tablet preparation. The results are shown in Table 36 and Table 37.

**Table 36 List of Formulations for Screening Filler Types and Amounts (1)**

| Material Name | | | Formulation Proportion/% | | | | | | |
|---|---|---|---|---|---|---|---|---|---|
| | | | Tablets Formulation for Screening | | | | | | |
| | | | Exam ple 64 | Exam ple 73 | Exam ple 74 | Exam ple 75 | Exam ple 76 | Exam ple 77 | Exam ple 78 |
| Tablet weight of 30 mg strength core tablet/mg | | | 500 | 500 | 500 | 500 | 500 | 500 | 500 |
| Intern al | Solid Dispersi ons | AP I | 6.0 | 6.0 | 6.0 | 6.0 | 6.0 | 6.0 | 6.0 |
| | | PV P K3 0 | 18.0 | 18.0 | 18.0 | 18.0 | 18.0 | 18.0 | 18.0 |
| | Lactose | | 39.0 | - | - | - | - | 19.5 | 39.0 |
| | Microcrystalli ne Cellulose | | 19.5 | 19.5 | 39.0 | 29.25 | - | - | - |
| | Mannitol | | - | 39.0 | 19.5 | 29.25 | 58.5 | - | - |
| | Anhydrous Calcium Hydrogen Phosphate | | - | - | - | - | - | 39.0 | 19.5 |
| | Sorbitol | | - | - | - | - | - | - | - |
| | Pregelatinized Starch | | 10.0 | 10.0 | 10.0 | 10.0 | 10.0 | 10.0 | 10.0 |
| | Crospovidone | | 2.5 | 2.5 | 2.5 | 2.5 | 2.5 | 2.5 | 2.5 |
| | Colloidal Silicon Dioxide | | 1.0 | 1.0 | 1.0 | 1.0 | 1.0 | 1.0 | 1.0 |
| | Magnesium Stearate | | 1.0 | 1.0 | 1.0 | 1.0 | 1.0 | 1.0 | 1.0 |
| Exter nal | Crospovidone | | 2.5 | 2.5 | 2.5 | 2.5 | 2.5 | 2.5 | 2.5 |
| | Magnesium Stearate | | 0.5 | 0.5 | 0.5 | 0.5 | 0.5 | 0.5 | 0.5 |
| Film Coating Premixes | | | 3.0 | - | - | - | - | - | - |

**Table 37 List of Formulations for Screening Filler Types and Amounts (2)**

| Material Name | | | Formulation Proportion/% | | | | | | |
|---|---|---|---|---|---|---|---|---|---|
| | | | Tablets Formulation for Screening | | | | | | |
| | | | Exam ple 64 | Exam ple 79 | Exam ple 80 | Exam ple 81 | Exam ple 82 | Exam ple 83 | Exam ple 84 |
| Tablet weight of 30 mg strength core tablet/mg | | | 500 | 500 | 500 | 500 | 500 | 500 | 500 |
| | Solid Dispersi ons | AP I | 6.0 | 6.0 | 6.0 | 6.0 | 6.0 | 6.0 | 6.0 |
| | | PV P K3 0 | 18.0 | 18.0 | 18.0 | 18.0 | 18.0 | 18.0 | 18.0 |
| | Lactose | | 39.0 | 29.25 | - | - | - | - | - |
| | Microcrystalli ne Cellulose | | 19.5 | - | - | 19.5 | 39.0 | 29.25 | - |
| | Mannitol | | - | - | - | - | - | - | - |
| Intern al | Anhydrous Calcium Hydrogen Phosphate | | - | 29.25 | 58.5 | - | - | - | - |
| | Sorbitol | | - | - | - | 39.0 | 19.5 | 29.25 | 58.5 |
| | Pregelatinized Starch | | 10.0 | 10.0 | 10.0 | 10.0 | 10.0 | 10.0 | 10.0 |
| | Crospovidone | | 2.5 | 2.5 | 2.5 | 2.5 | 2.5 | 2.5 | 2.5 |
| | Colloidal Silicon Dioxide | | 1.0 | 1.0 | 1.0 | 1.0 | 1.0 | 1.0 | 1.0 |
| | Magnesium Stearate | | 1.0 | 1.0 | 1.0 | 1.0 | 1.0 | 1.0 | 1.0 |
| | Crospovidone | | 2.5 | 2.5 | 2.5 | 2.5 | 2.5 | 2.5 | 2.5 |
| Exter nal | Magnesium Stearate | | 0.5 | 0.5 | 0.5 | 0.5 | 0.5 | 0.5 | 0.5 |
| Film Coating Premixes | | | 3.0 | - | - | - | - | - | - |

### 10.1.3.2.2 Preparation Process

The preparation processes for Examples 74-84 were the same as those for Examples 66-73.

### 10.1.3.2.3 Test Results

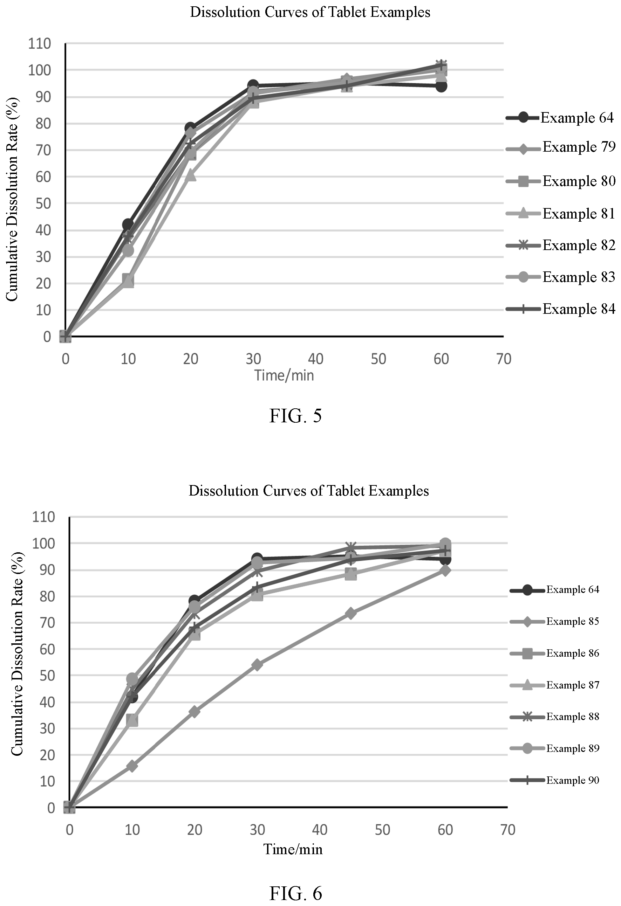

The dissolution results are shown in Table 38, Table 39, Figure 4, and Figure 5.

**Table 38 Summary of Dissolution Data for Tablet Formulations for Screening Filler Types and Addition Methods (1)**

| Time/mi n | Dissolution/% | | | | | | |
|---|---|---|---|---|---|---|---|
| | Exampl e 64 | Exampl e 73 | Exampl e 74 | Exampl e 75 | Exampl e 76 | Exampl e 77 | Exampl e 78 |
| | 30 mg Specification | | | | | | |
| 0 | 0 | 0 | 0 | 0 | 0 | 0 | 0 |
| 10 | 42 | 21.6 | 26.2 | 24.5 | 32.8 | 25.1 | 31.4 |
| 20 | 78 | 79.9 | 60.4 | 62.1 | 74.9 | 76.2 | 74.3 |
| 30 | 94 | 91.8 | 90.7 | 91.8 | 88.8 | 92.6 | 88.7 |
| 45 | 95 | 95.6 | 96.7 | 95.3 | 95.2 | 94.4 | 96.8 |
| 60 | 94 | 99.1 | 98.5 | 101.5 | 99.8 | 99.1 | 101 |

**Table 39 Summary of Dissolution Data for Tablet Formulations for Screening Filler Types and Addition Methods (2)**

| Time/mi n | Dissolution/% | | | | | | |
|---|---|---|---|---|---|---|---|
| | Exampl e 64 | Exampl e 79 | Exampl e 80 | Exampl e 81 | Exampl e 82 | Exampl e 83 | Exampl e 84 |
| | 30 mg Strength | | | | | | |
| 0 | 0 | 0 | 0.0 | 0.0 | 0.0 | 0.0 | 0.0 |
| 10 | 42 | 37.8 | 21.5 | 20.9 | 36.7 | 32.5 | 37.6 |
| 20 | 78 | 76.4 | 68.6 | 60.8 | 69.1 | 69.4 | 72.6 |
| 30 | 94 | 91.2 | 88.2 | 88.2 | 92 | 91.5 | 89.4 |
| 45 | 95 | 96.5 | 95.5 | 94.2 | 94.8 | 95.3 | 94 |
| 60 | 94 | 101 | 100.1 | 98 | 101.4 | 101.3 | 101.8 |

The dissolution method for the above examples was: paddle method at 75 rpm, 0.1 mol/L HCl + 0.1% Tween 80.

### 10.1.4 Examples for Tablet Disintegrant Screening

Four disintegrants-crospovidone (PVPP), pregelatinized starch (P.Starch), croscarmellose sodium (CC-Na), and sodium starch glycolate (CMS-Na)-and their addition methods were investigated.

### 10.1.4.1 Examples of Tablets Using Pregelatinized Starch as the Main Disintegrant

### 10.1.4.1.1 Formulation Composition

**Table 40 Investigation of Amount and Addition Methods for Disintegrant P. Starch**

| Material Name | | | Formulation Proportion/% | | | | | | | |
|---|---|---|---|---|---|---|---|---|---|---|
| | | | Tablets Formulation for Screening | | | | | | | |
| | | | Exa mple 64 | Exa mple 85 | Exa mple 86 | Exa mple 87 | Exa mple 88 | Exa mple 89 | Exa mple 90 | Exa mple 91 |
| Tablet weight of 30 mg strength core tablet/mg | | | 500 | 500 | 500 | 500 | 500 | 500 | 500 | 500 |
| Exter nal | Crospovido ne | | 2.5 | - | - | - | - | - | - | - |
| | Pregelatiniz ed Starch | | - | - | - | - | - | - | - | 5.0 |
| | Magnesium Stearate | | 0.5 | 0.5 | 0.5 | 0.5 | 0.5 | 0.5 | 0.5 | 0.5 |
| Film Coating Premixes | | | 3.0 | - | - | - | - | - | - | - |

### 10.1.4.1.2 Preparation Process

The preparation processes for Examples 85-91 were the same as those for Examples 66-73.

### 10.1.4.1.3 Test Results

The dissolution results are shown in Table 41 and Figure 6.

**Table 41 Summary of Dissolution Data for Tablet Formulations for Investigation of the Amount and Addition Methods of Disintegrant P.Starch**

| Time/m in | Dissolution/% | | | | | | | |
|---|---|---|---|---|---|---|---|---|
| | Examp le 64 | Examp le 85 | Examp le 86 | Examp le 87 | Examp le 88 | Examp le 89 | Examp le 90 | Examp le 91 |
| | 30 mg Strength | | | | | | | |
| 0 | 0 | 0 | 0 | 0 | 0 | 0 | 0 | 0 |
| 10 | 42 | 15.6 | 22.6 | 33.2 | 45 | 48.6 | 49.6 | 42.1 |
| 20 | 78 | 36.4 | 48.6 | 65.7 | 73.7 | 76.2 | 78.2 | 68.2 |
| 30 | 94 | 53.9 | 66.7 | 80.6 | 89.4 | 92.6 | 95.2 | 83.6 |
| 45 | 95 | 73.6 | 83.1 | 88.6 | 98.2 | 94.4 | 98.1 | 93.9 |
| 60 | 94 | 89.7 | 95.5 | 97.2 | 99.1 | 99.8 | 102.0 | 97.3 |

The dissolution method for the above examples was: paddle method at 75 rpm, 0.1 mol/L HCl + 0.1% Tween 80.

From Table 41, it can be seen that in the 0.1 mol/L HCl - 0.1% Tween 80 medium, the dissolution rate increases with the increase in the amount of P.Starch .

### 10.1.4.2 Examples of Tablets Using Crospovidone as Disintegrant

### 10.1.4.2.1 Formulation Composition

**Table 42 Investigation of Amount and Addition Methods for Disintegrant Crospovidone**

| Material Name | | | Formulation Proportion/% | | | | | | |
|---|---|---|---|---|---|---|---|---|---|
| | | | Tablets Formulation for Screening | | | | | | |
| | | | Exam ple 64 | Exam ple 92 | Exam ple 93 | Exam ple 94 | Exam ple 95 | Exam ple 96 | Exam ple 97 |
| Tablet weight of 30 mg strength core tablet/mg | | | 500 | 500 | 500 | 500 | 500 | 500 | 500 |
| Intern al | Solid Dispersi ons | AP I | 6.0 | 6.0 | 6.0 | 6.0 | 6.0 | 6.0 | 6.0 |
| | | PV P K3 0 | 18.0 | 18.0 | 18.0 | 18.0 | 18.0 | 18.0 | 18.0 |
| | Lactose | | 39.0 | 46.0 | 45.0 | 44.0 | 42.0 | 41.0 | 32.0 |
| | Microcrystalli ne Cellulose | | 19.5 | 23.5 | 22.5 | 21.50 | 21.50 | 20.5 | 31.5 |
| | Pregelatinized Starch | | 10.0 | - | - | - | - | - | - |
| | Crospovidone | | 2.5 | 4.0 | 6.0 | 8.0 | 10.0 | 12.0 | 5.0 |
| | Colloidal Silicon Dioxide | | 1.0 | 1.0 | 1.0 | 1.0 | 1.0 | 1.0 | 1.0 |
| | Magnesium Stearate | | 1.0 | 1.0 | 1.0 | 1.0 | 1.0 | 1.0 | 1.0 |
| Exter nal | Crospovidon e | | 2.5 | - | - | - | - | - | 5.0 |
| | Magnesium Stearate | | 0.5 | 0.5 | 0.5 | 0.5 | 0.5 | 0.5 | 0.5 |

### 10.1.4.2.2 Preparation Process

The preparation processes for Examples 85-91 were the same as those for Examples 66-73.

### 10.1.4.2.3 Test Results

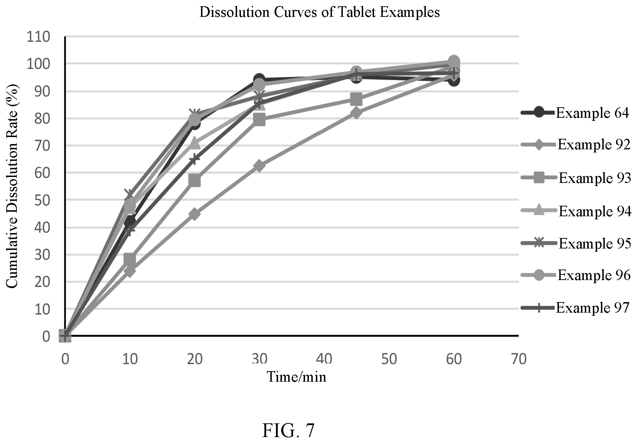

The dissolution results are shown in Table 43 and Figure 7.

**Table 43 Summary of Dissolution Data for Tablet Formulations for Investigation of the Amount and Addition Methods of Disintegrant Crospovidone**

| Time/mi n | Dissolution/% | | | | | | |
|---|---|---|---|---|---|---|---|
| | Exampl e 64 | Exampl e 92 | Exampl e 93 | Exampl e 94 | Exampl e 95 | Exampl e 96 | Exampl e 97 |
| | 30 mg Specification | | | | | | |
| 0 | 0 | 0 | 0 | 0 | 0 | 0 | 0 |
| 10 | 42 | 24 | 28.1 | 47.3 | 51.9 | 48.1 | 38.9 |
| 20 | 78 | 45 | 57.1 | 71 | 81.3 | 79.4 | 64.9 |
| 30 | 94 | 62.4 | 79.4 | 85.1 | 88.2 | 92.2 | 85.6 |
| 45 | 95 | 82.1 | 86.9 | 96.3 | 95.9 | 96.8 | 96.3 |
| 60 | 94 | 96 | 99 | 100 | 99.6 | 101 | 96.6 |

The dissolution method for the above examples was: paddle method at 75 rpm, 0.1 mol/L HCl + 0.1% Tween 80.

From Table 43, it can be seen that in the 0.1 mol/L HCl - 0.1% Tween 80 medium, the dissolution rate increases with the increase in the amount of crospovidone.

### 10.1.5 Examples for Tablet Formulation Antioxidant Screening

The compound of the present invention (API) is prone to oxidation. During the preparation of the formulation, a certain amount of antioxidant can be added to slow down the oxidation reaction. There are two methods for adding antioxidants: one is to add it to the first pharmaceutical composition, i.e., the solid dispersion; the other is to add it to the excipients of the second pharmaceutical composition excluding the first pharmaceutical composition.

### 10.1.5.1 Examples of Solid Dispersions Containing Antioxidants

### 10.1.5.1.1 The formulations are shown in Table 44.

**Table 44 Summary of Formulations**

| Example | | Example 98 | Example 99 | Example 100 |
|---|---|---|---|---|
| Material Name | | w/w (%) | | |
| Solid | Compound of the Invention (API) | 20 | 20 | 20 |
| | PVP K30 | 80 | 79.98 | 79.6 |
| | BHA | - | 0.01 | - |
| | BHT | - | 0.01 | - |
| | Sodium Metabisulfite | - | - | 0.4 |
| | Total | 100 | 100 | 100 |
| Solvent | Acetone: Methanol 2:1 | Appropriate Amount | Appropriate Amount | Appropriate Amount |
| Solid Content | | 10% | 10% | 10% |

### 10.1.5.1.2 Preparation Process:

Drug Solution Preparation: Prepare a mixed solvent of acetone:methanol = 2:1 (w/w). Dissolve the API, povidone, and antioxidant in the acetone:methanol = 2:1 (w/w) mixed solvent, and stir until the solution becomes clear.

Spray Drying: Set the inlet air temperature to 85°C, outlet air temperature to 50°C, and atomizing gas flow rate to 5.5 kg/h (3.0 kg/h to 12.0 kg/h). Spray the drug solution to prepare the solid dispersion.

Vacuum Drying: Set the vacuum drying oven temperature to 60°C, and vacuum degree to ≤ -0.1 MPa, and dry for 12 to 48 hours (until residual solvent testing meets standards).

### 10.1.5.1.3 Test Results

The prepared Examples 98-100 were stored under conditions of 40°C/75% RH, and samples were taken at day 10 and day 30 to test related substances, with a focus on the level of oxidative impurities. The experimental results showed that the oxidative impurities in Example 98 increased from 0.20% at day 0 to 0.33% at day 10 and 0.41% at day 30, indicating significant growth. The oxidative impurities in Example 99 increased from 0.12% at day 0 to 0.15% at day 10 and 0.20% at day 30. Compared with the sample of Example 98 without antioxidants, the extent of increase was significantly reduced, and the level at day 0 was lower than that of Example 98, indicating that the addition of antioxidants BHA and BHT (ratio 1:1, total amount 0.02% (percentage of the total solid dispersion)) to the SDD exerted a clear antioxidant effect on this sample. The oxidative impurities in Example 100 (API:PVP K30 = 1:4, with antioxidant sodium metabisulfite added at 0.4% (percentage of the total solid dispersion)) remained at 0.01% at day 0, day 10, and day 30, indicating that the antioxidant sodium metabisulfite exerted a significant antioxidant effect on this SDD sample.

Therefore, adding antioxidants such as BHA, BHT, sodium metabisulfite, etc. (used alone or in combination) to the solid dispersion formulation can significantly inhibit the growth of oxidative impurities. An antioxidant addition amount of 0.01% to 0.5% (percentage of the total solid dispersion) is sufficient to exert an antioxidant effect. Other antioxidants such as Vitamin E, Vitamin E Acetate, L-ascorbyl palmitate (L-AP), etc., can also provide similar antioxidant effects.

### 10.1.5.2 Examples of Tablets Containing Antioxidants

### 10.1.5.2.1 Formulation Composition of Examples of Tablets Containing Antioxidants

**Table 45 List of Formulations for Examples of Tablets Containing Antioxidants**

| Material Name | | | Example 65 | | Example 101 | | Example 102 | |
|---|---|---|---|---|---|---|---|---|
| | | | Formulati on Composit ion% | mg/ta blet | Formulati on Composit ion% | mg / tabl et | Formulati on Composit ion% | mg / tabl et |
| Core Tablet Composition | | | | | | | | |
| | Solid Dispers ions | API | 6.0 | 30.0 | 6.0 | 30. 0 | 6.0 | 30. 0 |
| | | PVP K30 | 17.904 | 89.5 | 17.9904 | 89. 95 | 18.0 | 90. 0 |
| | | Sodium Metabis ulfite | 0.096 | 0.48 | 0.0096 | 0.0 48 | - | - - |
| | Lactose | | 39.0 | 195.0 | 39.0 | 195 .0 | 39.0 | 195 .0 |
| Inter nal | Microcrystalline Cellulose | | 19.5 | 97.5 | 19.5 | 97. 5 | 19.5 | 97. 5 |
| | Pregelatinized Starch | | 10.0 | 50.0 | 10.0 | 50. 0 | 10.0 | 50. 0 |
| | Crospovidone | | 2.5 | 12.5 | 2.5 | 12. 5 | 2.5 | 12. 5 |
| | Colloidal Silicon Dioxide | | 1.00 | 5.0 | 1.00 | 5.0 | 1.00 | 5.0 |
| | Magnesium Stearate | | 1.00 | 5.0 | 1.00 | 5.0 | 1.00 | 5.0 |
| Exter nal | Crospovidone | | 2.5 | 12.5 | 2.5 | 12. 5 | 2.5 | 12. 5 |
| | Magnesium Stearate | | 0.5 | 2.5 | 0.5 | 2.5 | 0.5 | 2.5 |
| Total | | | 100 | 500 | 100 | 500 | 100 | 500 |

| Coating | | | | | | | | |
|---|---|---|---|---|---|---|---|---|
| Film Coating Premixes | 3.0 | 15.0 | 3.0 | 15. 0 | 3.0 | | 15. 0 | |

### 10.1.5.2.2 Preparation Process

The preparation processes for Example 101 and Example 102 were the same as that for Example 65.

### 10.1.5.2.3 Investigation of Changes in Oxidative Impurities in Tablets

The samples were stored under conditions of 40°C/75% RH, packaged and sealed in HDPE bottles. By determining the related substances of the samples at different time points, the changes in oxidative impurities are summarized in Table 46:

**Table 46 Changes in Oxidative Impurities in Tablets Over Time**

| Example | Oxidative Impurities% | | | | |
|---|---|---|---|---|---|
| | 0 | 1M | 2M | 3M | 6M |
| Example 65 | 0.01 | 0.01 | 0.01 | 0.01 | 0.01 |
| Example 101 | 0.01 | 0.01 | 0.02 | 0.04 | 0.05 |
| Example 102 | 0.08 | 0.14 | 0.22 | 0.28 | 0.33 |

Examples 65, 101, and 102 were prepared into tablets using the same batch of API. When the amount of the antioxidant sodium metabisulfite added to the formulation exceeded 0.0096%, significant antioxidant effects were observed.

### 10.2 Capsule Examples

### 10.2.1 Formulations

**Table 47 List of Example Formulations**

| Material Name | | | Example 103 | |
|---|---|---|---|---|
| | | | Formulation Composition% | mg/tablet |
| Internal | Solid Dispersions | API | 8.0 | 40.0 |
| | | Copovidone S630 | 32.0 | 160.0 |
| | Lactose | | 29.0 | 145.0 |
| | Microcrystalline Cellulose | | 14 | 70.0 |
| | Croscarmellose Sodium | | 9.0 | 45.0 |
| | Sodium Lauryl Sulfate | | 6.0 | 30.0 |
| | Sodium Metabisulfite | | 1.0 | 5.0 |
| External | Magnesium Stearate | | 1.0 | 5.0 |
| Total | | | 100.0 | 500 |

### Preparation Process:

### (1) Preparation of Solid Dispersion

Thoroughly mix the API and excipients. Set the hot melt extrusion temperature to 160-175°C, adjust the feeding speed to 5-20 rpm, and the screw rotation speed to 20-80 rpm to perform hot melt extrusion. After extrusion, cool the material, then mill it, and sieve it through a 40-mesh sieve for later use.

### (2) Preparation of the Second Pharmaceutical Composition

Material Preparation: Weigh the internal excipients lactose, microcrystalline cellulose, and croscarmellose sodium according to the formula amounts. Weigh sodium metabisulfite and dissolve it in purified water to prepare a 2% (w/w) aqueous solution of sodium metabisulfite.

Preparation of Internal Granules Containing Sodium Metabisulfite by Fluidized Bed One-Step Granulation: Add the formula amounts of lactose, microcrystalline cellulose, croscarmellose sodium, and sodium lauryl sulfate to the fluidized bed. Set the fluidized bed inlet air temperature to 45-75°C, the fluidized bed material temperature to 35-55°C, and adjust the inlet air volume to 15-80 m³/h, spray rate to 5-50 g/min, and atomization pressure to 0.05-1.0 bar. Perform fluidized bed one-step granulation, and dry for 10-30 minutes after spraying was completed.

Final Blending: Calculate the weight of the internal granules containing sodium metabisulfite based on the weight of the solid dispersion, and weigh accordingly. Add the solid dispersion and the internal granules containing sodium metabisulfite to a blender bin. Set the blending speed to 15 rpm and blend for 5-8 minutes. Calculate and weigh the external excipients, add them to the above mixture, set the blending speed to 15 rpm, and blend for 2-3 minutes.

Capsule Filling: Take the final mixture and use a capsule filling machine to fill hard gelatin capsules, selecting size 00 capsules.

### 10.3 Granule Examples

### 10.3.1 Formulation Composition

**Table 48 Formulation Composition Table**

| Excipient Name | Example 104 | |
|---|---|---|
| | Formulation Composition/% | Batch Formula (g) |
| Compound of the Invention (API) | 8.0 | 80.0 |
| HPC EXF | 24.0 | 240.0 |
| Acetone | / | 4053.3 |
| Methanol | / | 2026.7 |
| Lactose | 38.0 | 380.0 |
| Microcrystalline Cellulose | 20.0 | 200.0 |
| Sodium Starch Glycolate | 8.0 | 80.0 |
| Sodium Lauryl Sulfate | 1.0 | 10.0 |
| Magnesium Stearate | 1.0 | 10.0 |

### 10.3.2 Preparation Process:

Drug Solution Preparation: Dissolve the API and carrier excipients in a mixed solvent of acetone:methanol = 2:1, and stir until completely dissolved to obtain a drug solution with a solid content of 5% for later use.

Fluidized Bed Granulation: Add lactose, microcrystalline cellulose, sodium starch glycolate, and sodium lauryl sulfate to the fluidized bed. Set the fluidized bed inlet air temperature to 45-75°C, the fluidized bed material temperature to 35-55°C, and adjust the inlet air volume to 15-80 m³/h, spray rate to 5-50 g/min, and atomization pressure to 0.05-1.0 bar. Perform fluidized bed one-step granulation, dry for 10-30 minutes after spraying was completed, and then discharge the material.

Final Blending: Sieve the granules obtained from fluidized bed granulation, and blend with magnesium stearate using a bin blender for 2-3 minutes to obtain the final blend.

Granule Packaging: Package the final blend into aluminum foil bags, seal, and obtain the granule form of the second pharmaceutical composition.

### 10.4 Examples of Preparing Second Pharmaceutical Composition by Fluidized Bed One-Step Granulation

Examples of preparing the second pharmaceutical composition using fluidized bed granulation (top-spray granulation method)

### 10.4.1 Formulation Composition of Examples 105-106 (taking a batch size of 1000 g as an example)

**Table 49 Summary of Formulation Composition**

| Excipient Name | Example 105 | | Example 106 | |
|---|---|---|---|---|
| | Formulation Composition/% | Batch Formula (g) | Formulation Composition/% | Batch Formula (g) |
| Compound of the Invention (API) | 6.0 | 60.0 | 6.0 | 60.0 |
| Povidone K30 | 17.904 | 179.04 | - | - |
| Copovidone S630 | - | - | 23.904 | 239.04 |
| Sodium Metabisulfite | 0.096 | 0.96 | 0.096 | 0.96 |
| Acetone | - | 1440.0 | - | 1800.0 |
| Methanol | - | 720.0 | - | 900.0 |
| Purified Water | - | 3.0 | - | 3.0 |
| Lactose | 39.0 | 390.0 | 35.0 | 350.0 |
| Microcrystalline Cellulose | 19.5 | 195.0 | 17.5 | 175.0 |
| Pregelatinized Starch | 12.5 | 125.0 | 12.5 | 125.0 |
| Colloidal Silicon Dioxide | 1.0 | 10.0 | 1.0 | 10.0 |
| Magnesium Stearate (Internal) | 1.0 | 10.0 | 1.0 | 10.0 |
| Crospovidone (External) | 2.5 | 25.0 | 2.5 | 25.0 |
| Magnesium Stearate (External) | 0.5 | 5.0 | 0.5 | 5.0 |

### 10.4.2 Preparation Process:

### (1) Fluidized Bed One-Step Granulation:

Prepare the drug solution: Dissolve the API and carrier excipients in a mixed solvent of acetone:methanol = 2:1. Dissolve sodium metabisulfite in 3 times the amount of purified water and add it to the above solution. Mix uniformly to obtain a drug solution with a solid content of 10% for later use.

Fluidized bed granulation: Add the fillers lactose, microcrystalline cellulose, and pregelatinized starch to the fluidized bed. Set the fluidized bed inlet air temperature to 45-75°C, the fluidized bed material temperature to 35-55°C, and adjust the inlet air volume to 15-80 m³/h, spray rate to 5-50 g/min, and atomization pressure to 0.05-1.0 bar. Perform fluidized bed one-step granulation, dry for 10-30 minutes after spraying was completed, and discharge the material.

Dry Granulation: Sieve the granules obtained from fluidized bed granulation, and blend with colloidal silicon dioxide and magnesium stearate (internal) using a bin blender. Use a dry granulator to perform dry granulation on the mixed granules. Set the dry granulation parameters as follows: hydraulic pressure 20 bar (18-230 bar), roller unit speed 8 rpm (3.4-14.8 rpm), roller gap 1.5 mm, primary sizing speed 40 rpm, and secondary sizing speed 50 rpm (24.9-108.4 rpm) for granulation.

Final blending: Calculate and weigh the external excipients based on the weight of the granules after dry granulation. Blend the granules after dry granulation and the external excipients (excluding magnesium stearate) using a bin blender. Set the blending speed to 15 rpm and blend for 5 minutes. Add magnesium stearate (external), set the blending speed to 15 rpm, and blend for 2 minutes.

Capsule Filling: Fill the final blend into hard gelatin capsules or hypromellose hard capsules to obtain the capsule form of the second pharmaceutical composition.

### 11. Animal PK Experiments

### 11.1 Comparison of Bioavailability of Solid Dispersions and API in Dogs

Based on the solubility data from the solid dispersion formulation examples above, the API (administration form: 10 mg/mL suspension, API suspended in 0.5% CMC-Na solution) and solid dispersions prepared using PVP K30 (drug-excipient ratios of 1:3 and 1:4, respectively) were selected for dog PK experiments. The solid dispersions were filled into hard capsules and orally administered to beagle dogs (male, fasted, 3 dogs per group) at a dose of 5 mg/kg. Whole blood samples of 0.3 mL were collected at 0 min, 10 min, 15 min, 30 min, 1 h, 2 h, 3 h, 4 h, 6 h, 8 h, 12 h, and 24 h after administration. The blood samples were placed in anticoagulant tubes, gently inverted several times, and then kept on wet ice in preparation for centrifugation. Within 30 minutes after collection, the samples were centrifuged at about 2000 × g (force) at 4°C for 10 minutes to obtain plasma. The resulting plasma samples were immediately divided into two aliquots (about 75 µL each) and transferred to cryovials. The samples were maintained on wet ice throughout the processing and stored in a freezer at -75 ± 15°C until analysis. The concentrations of the test substance in the plasma samples were analyzed using an LC-MS/MS method.

**Table 50 Summary of Dog PK Data for SDD Formulations**

| | Solid Dispersion Powder With Drug-Excipient Ratio of 1:3 (Filled in Capsules), Corresponding to Example 4 | Solid Dispersion Powder With Drug-Excipient Ratio of 1:4 (Filled in Capsules), Corresponding to Example 5 | API (Administration Form: 10 mg/mL Suspension, API Suspended in 0.5% CMC-Na Solution) |
|---|---|---|---|
| Dose (mg/kg) | 5 | 5 | 5 |
| Cₘₐₓ (ng/mL) | 59.7 | 41.0 | 22.0 |
| AUC (h*ng/mL) | 233 | 162 | 59 |

The AUC and Cₘₐₓ data show significant differences in the exposure of the active substance after oral administration of the solid dispersions and the API: at an administration dose of 5 mg/kg, the AUC in animals administered SDD increased by 2 to 3 times, and Cₘₐₓ increased by 1 to 2 times, demonstrating that the formation of solid dispersions can significantly improve the oral absorption and exposure of the active substance.

### 11.2 Dog PK Experiment for Tablets

The tablet formulations of Example 59, Example 60, and Example 65 were selected for dog PK experiments.

Example 59 and Example 60 were orally administered to beagle dogs (male, fasted, 3 dogs per group) at a dose of about 5 mg/kg. Example 65 was orally administered to beagle dogs (half male and half female, fasted, 8 dogs) at a dose of 50 mg/animal (about 5 mg/kg). Whole blood samples of 0.3 mL were collected at 0 min, 10 min, 15 min, 30 min, 1 h, 2 h, 3 h, 4 h, 6 h, 8 h, 12 h, and 24 h after administration. The blood samples were placed in anticoagulant tubes, gently inverted several times, and then kept on wet ice in preparation for centrifugation. Within 30 minutes after collection, the samples were centrifuged at about 2000 × g (force) at 4°C for 10 minutes to obtain plasma. The resulting plasma samples were immediately divided into two aliquots (about 75 µL each) and transferred to cryovials. The samples were maintained on wet ice throughout the processing and stored in a freezer at -75 ± 15°C until analysis. The concentrations of the test substance in the plasma samples were analyzed using an LC-MS/MS method.

**Table 51 Summary of Dog PK Data for Tablets**

| | Example 59 Tablet Specification: 22.5 mg/tablet | Example 60 Tablet Specification: 22.5 mg/tablet | Example 65 Tablet Specification: 30 mg/tablet | API (Administration Form: 10 mg/mL suspension, API suspended in 0.5% CMC-Na solution) |
|---|---|---|---|---|
| Dose (mg/kg) | 5 mg/kg | 5 mg/kg | 50 mg/animal (about 5 mg/kg) | 5 mg/kg |
| Cₘₐₓ (ng/mL) | 52.5 | 55.5 | 69.4 | 22.0 |
| AUC (h*ng/mL) | 169 | 183 | 218.8 | 59 |

The AUC and Cₘₐₓ data indicate significant differences in the exposure of the active substance after oral administration of the tablet formulations and the API: at an administration dose of 5 mg/kg or 50 mg/animal, the AUC of the tablets increased by 2 to 3 times, and Cₘₐₓ increased by 1.5 to 2.5 times, demonstrating that the tablet formulations prepared using solid dispersions can significantly improve the oral absorption and exposure of the active substance.

The embodiments of the present invention have been described in detail above. Specific examples are used in this document to explain the principles and implementation of the present invention, and the descriptions of the above examples are only intended to help understand the methods and core concepts of the present invention. At the same time, changes or modifications made by those skilled in the art based on the concepts of the present invention, in terms of specific embodiments and scope of application, fall within the scope of protection of the present invention. In summary, the content of this specification should not be construed as limiting the present invention.

## Claims

1. A first pharmaceutical composition containing a substituted indol-5-ol derivative, wherein the first pharmaceutical composition contains
(a) a therapeutically effective amount of a compound of formula (I), or a pharmaceutically acceptable salt thereof; and 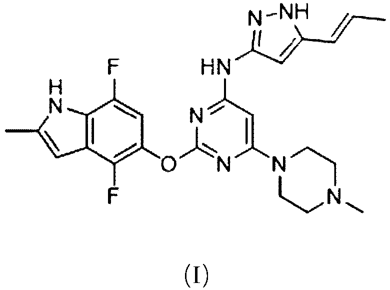
(b) one or more polymer materials, such as 1, 2, 3, 4, or 5 polymer materials.

2. The first pharmaceutical composition according to claim 1, wherein the polymer material is selected from one or more of the following: hypromellose acetate succinate, hydroxyalkyl cellulose, povidone, copovidone, polypropylene resin, polyvinyl caprolactam-polyvinyl acetate-polyethylene glycol graft copolymer, and polyethylene glycol;
preferably, the hypromellose acetate succinate is hypromellose acetate succinate AS-MMP and/or hypromellose acetate succinate AS-MF;
preferably, the hydroxyalkyl cellulose is hydroxypropyl cellulose, hypromellose, and/or hydroxyethyl cellulose;
preferably, the hydroxypropyl cellulose is hydroxypropyl cellulose EXF, hydroxypropyl cellulose HXF, and/or hydroxypropyl cellulose LXF;
preferably, the hypromellose is hypromellose E3, hypromellose E5, and/or hypromellose E15;
preferably, the povidone is povidone K25, povidone K30, povidone K-12, and/or povidone K-17;
preferably, the copovidone is copovidone S630;
preferably, the polypropylene resin is polypropylene resin E100;
preferably, the polyethylene glycol is polyethylene glycol 4000 and/or polyethylene glycol 6000;
also preferably, the polymer material is povidone K30, hypromellose E3, hypromellose E15, hypromellose acetate succinate AS-MMP, hydroxypropyl cellulose EXF, copovidone S630, polyethylene glycol 4000, polyvinyl caprolactam-polyvinyl acetate-polyethylene glycol graft copolymer, or polypropylene resin E100;
more preferably, a mass ratio of the compound of formula (I) to the polymer material is 1:1 to 1:10;
more preferably, the mass ratio of the compound of formula (I) to the polymer material is 1:1 to 1:6;
more preferably, the mass ratio of the compound of formula (I) to the polymer material is 1:3 to 1:6.

3. The first pharmaceutical composition according to claim 1 or 2, wherein the first pharmaceutical composition further contains one or more antioxidants, such as 1, 2, 3, 4, or 5 antioxidants;
preferably, the antioxidant is selected from one or more of the following: butylated hydroxyanisole, butylated hydroxytoluene, sodium metabisulfite, vitamin E, vitamin E acetate, and L-ascorbyl palmitate;
more preferably, the antioxidant is sodium metabisulfite;
also preferably, the antioxidant is present in an amount of 0.01% to 3% such as about 0.2% based on a weight of the first pharmaceutical composition;
especially preferably, the first pharmaceutical composition is in a form of a solid dispersion.

4. A second pharmaceutical composition comprising the first pharmaceutical composition according to any one of claims 1 to 3, wherein the second pharmaceutical composition further contains
(1) one or more fillers, such as 1, 2, 3, 4, or 5 fillers;
(2) one or more disintegrants, such as 1, 2, 3, 4, or 5 disintegrants;
(3) optionally one or more surfactants, such as 1, 2, 3, 4, or 5 surfactants;
(4) optionally one or more glidants, such as 1, 2, 3, 4, or 5 glidants;
(5) optionally one or more lubricants, such as 1, 2, 3, 4, or 5 lubricants; and
(6) optionally one or more film coating premixes, such as 1, 2, 3, 4, or 5 film coating premixes;
preferably, the filler is selected from one or more of the following: lactose (such as lactose monohydrate or anhydrous lactose), starch, calcium hydrogen phosphate (such as anhydrous calcium hydrogen phosphate or calcium hydrogen phosphate dihydrate), calcium sulfate dihydrate, mannitol, sorbitol, and microcrystalline cellulose;
preferably, the microcrystalline cellulose is microcrystalline cellulose PH101;
preferably, the disintegrant is selected from one or more of the following: croscarmellose sodium, crospovidone (such as crospovidone XL-10, crospovidone CL), pregelatinized starch, and sodium starch glycolate;
preferably, the surfactant is sodium lauryl sulfate;
preferably, the glidant is colloidal silicon dioxide;
preferably, the lubricant is magnesium stearate and/or glyceryl tribehenate;
more preferably, the filler is present in an amount of 30% to 70% based on a weight of the second pharmaceutical composition;
more preferably, the filler is present in an amount of 55% to 65% such as about 58.5% based on the weight of the second pharmaceutical composition;
more preferably, the disintegrant is present in an amount of 6% to 20% based on the weight of the second pharmaceutical composition;
more preferably, the disintegrant is present in an amount of 10% to 20% such as about 15% based on the weight of the second pharmaceutical composition;
more preferably, the surfactant is present in an amount of 1% to 6% based on the weight of the second pharmaceutical composition;
more preferably, the glidant is present in an amount of 0.5% to 3% such as about 1% based on the weight of the second pharmaceutical composition;
more preferably, the lubricant is present in an amount of 0.5% to 3% such as about 1.5% based on the weight of the second pharmaceutical composition;
more preferably, the film coating premix is present in an amount of 0.5% to 5% such as about 3% based on the weight of the second pharmaceutical composition;
more preferably, the antioxidant is present in an amount of 0.01% to 1% such as about 0.1% based on the weight of the second pharmaceutical composition;
also preferably, the second pharmaceutical composition is in a form of an oral preparation;
also preferably, the second pharmaceutical composition is in a form of a solid oral preparation, such as a tablet or a capsule.

5. A method for preparing the first pharmaceutical composition according to any one of claims 1 to 3, wherein the method comprises the following steps:
(a) mixing and dissolving the compound of formula (I) or the pharmaceutically acceptable salt thereof with the one or more polymer materials, such as 1, 2, 3, 4, or 5 polymer materials, one or more solvents, such as 1, 2, 3, 4, or 5 solvents, and optionally the one or more antioxidants, such as 1, 2, 3, 4, or 5 antioxidants, to form a mixed solution;
(b) spray drying the mixed solution to obtain a mixed powder; and
(c) vacuum drying or double cone drying the mixed powder to obtain the first pharmaceutical composition;
preferably, the solvent is selected from one or more of the following: water, methanol, acetone, ethanol, isopropanol, acetonitrile, ethyl acetate, propylene glycol, and dichloromethane;
also preferably, the solvent is selected from one or more of the following: water, methanol, ethanol, acetone, and dichloromethane;
also preferably, the solvent is water, methanol, ethanol, acetone, and/or dichloromethane;
also preferably, a mass ratio of the acetone to the methanol is 1:1 to 3:1, such as about 2:1;
also preferably, the solvent is about 70% acetone and methanol, and about 30% water;
also preferably, the solvent is about 40% acetone and methanol, and about 60% dichloromethane;
also preferably, a mass ratio of the solvent to the first pharmaceutical composition is 85:15 to 97:3;
also preferably, the mass ratio of the solvent to the first pharmaceutical composition is 90:10 to 94:6;
more preferably, conditions for the spray drying are as follows: setting an inlet air temperature of 70°C to 90°C, an outlet air temperature of 45°C to 55°C, a pump power of 20% to 45%, a condenser temperature of -15°C to -5°C, and an atomizing gas flow rate of 3 kg/h to 12 kg/h;
more preferably, conditions for the vacuum drying are as follows: after a vacuum degree stabilizes at ≤ -0.1 MPa, drying at 55°C to 65°C for 12 h to 48 h;
more preferably, conditions for the double cone drying are as follows: after a vacuum degree stabilizes at ≤ -0.1 MPa, drying at 55°C to 65°C for 4 h to 24 h.

6. A method for preparing the first pharmaceutical composition according to any one of claims 1 to 3, wherein the method comprises the following steps:
(a) sieving and uniformly mixing the compound of formula (I) or the pharmaceutically acceptable salt thereof with the one or more polymer materials, such as 1, 2, 3, 4, or 5 polymer materials, and optionally one or more plasticizers, such as 1, 2, 3, 4, or 5 plasticizers to obtain a mixture; and
(b) setting temperatures of segments of a screw of a hot melt extruder, adjusting a feeding speed to 5 rpm to 20 rpm and a screw rotation speed to 20 rpm to 80 rpm to perform hot melt extrusion, optionally cooling and pulverizing, to obtain the first pharmaceutical composition;
preferably, the plasticizer is selected from one or more of the following: dibutyl sebacate, diethyl phthalate, Poloxamer F-68, Poloxamer F-127, glyceryl monostearate, polyethylene glycol, sodium lauryl sulfate, dioctyl sodium sulfosuscinate, triethyl citrate, triacetin, Tween 80, and Vitamin E-TPGS;
also preferably, the temperatures are an initial segment temperature of 20°C to 40°C, a conveying segment temperature of 110°C to 130°C, a mixing segment temperature of 160°C to 180°C, a kneading segment temperature of 160°C to 180°C, and a conveying and discharging segment temperature of 150°C to 170°C.

7. A method for preparing the first pharmaceutical composition according to any one of claims 1 to 3, wherein the method comprises the following steps:
(a) mixing and dissolving the compound of formula (I) or the pharmaceutically acceptable salt thereof with the one or more polymer materials, such as 1, 2, 3, 4, or 5 polymer materials, and one or more solvents, such as 1, 2, 3, 4, or 5 solvents to form a mixed solution; and
(b) removing the solvent using a rotary evaporator at 45°C to 65°C, milling the mixture after rotary drying, and sieving through an 18-mesh to 80-mesh sieve to obtain the first pharmaceutical composition;
preferably, the solvent is selected from one or more of the following: water, methanol, acetone, ethanol, isopropanol, acetonitrile, ethyl acetate, propylene glycol, and dichloromethane;
also preferably, the solvent is selected from one or more of the following: water, methanol, ethanol, acetone, and dichloromethane;
also preferably, the solvent is water, ethanol, acetone, and/or dichloromethane;
also preferably, a mass ratio of the acetone to the methanol is 1:1 to 3:1, such as about 2:1;
also preferably, the solvent is about 70% acetone and methanol, and about 30% water;
also preferably, the solvent is about 40% acetone and methanol, and about 60% dichloromethane;
also preferably, a mass ratio of the solvent to the first pharmaceutical composition is 85:15 to 97:3;
also preferably, the mass ratio of the solvent to the first pharmaceutical composition is 90:10 to 94:6.

8. A method for preparing the second pharmaceutical composition according to claim 4, wherein the method comprises the following steps: spraying a mixed solution of the first pharmaceutical composition according to any one of claims 1 to 3 onto an appropriate amount of filler and/or disintegrant, drying and sieving to obtain a first pharmaceutical mixture; and mixing the first pharmaceutical mixture with a suitable filler, disintegrant, lubricant, and the like, to prepare the second pharmaceutical composition;
preferably, a dosage form of the second pharmaceutical composition is a tablet, a capsule, or a granule;
preferably, the method comprises the following steps:
(a) mixing and dissolving the compound of formula (I) or the pharmaceutically acceptable salt thereof with the one or more polymer materials, such as 1, 2, 3, 4, or 5 polymer materials, one or more solvents, such as 1, 2, 3, 4, or 5 solvents, and optionally the one or more antioxidants, such as 1, 2, 3, 4, or 5 antioxidants, to form a mixed solution;
(b) adding the filler and the disintegrant to a fluidized bed, and preheating;
(c) spraying the mixed solution of the first pharmaceutical composition into the fluidized bed, drying for 10 to 30 min, and sieving obtained granules;
(d) mixing the sieved granules with an appropriate amount of filler, disintegrant, glidant, and/or lubricant to obtain a second pharmaceutical mixture; and
(e) optionally, dispensing and tableting the second pharmaceutical mixture to obtain a tablet of the second pharmaceutical composition;
(f) optionally, dispensing and filling capsules with the second pharmaceutical mixture to obtain a capsule of the second pharmaceutical composition;
(g) optionally, dispensing and bagging the second pharmaceutical mixture to obtain a granule of the second pharmaceutical composition;
preferably, conditions for one-step granulation comprise a fluidized bed inlet air temperature of 45°C to 75°C, a fluidized bed material temperature of 35°C to 55°C, a fluidized bed inlet air volume of 15 m³/h to 80 m³/h, a fluidized bed spray rate of 5 g/min to 50 g/min, and a fluidized bed atomization pressure of 0.05 bar to 1.0 bar.

9. A method for preparing the second pharmaceutical composition according to claim 4, wherein the method comprises the following steps:
(a) performing a first blending of the first pharmaceutical composition with an appropriate amount of filler, disintegrant, glidant, and lubricant to obtain a first pharmaceutical mixture;
(b) performing dry granulation on the first pharmaceutical mixture to obtain granules;
(c) performing a second blending of the granules with an appropriate amount of disintegrant and lubricant to obtain a second pharmaceutical mixture; and
(d) optionally, dispensing and tableting the second pharmaceutical mixture to obtain a tablet of the second pharmaceutical composition;
(e) optionally, dispensing and filling capsules with the second pharmaceutical mixture to obtain a capsule of the second pharmaceutical composition;
(f) optionally, dispensing and bagging the second pharmaceutical mixture to obtain a granule of the second pharmaceutical composition;
preferably, a rotational speed for the first blending is 10 rpm to 20 rpm, and a blending time is 10 min to 15 min;
preferably, a rotational speed for the second blending is 10 rpm to 20 rpm, and a blending time is 2 min to 10 min;
more preferably, in the step (a), the disintegrant is crospovidone and pregelatinized starch;
more preferably, in the step (c), the disintegrant is crospovidone;
more preferably, a mass ratio of the crospovidone added in the step (a) to the crospovidone added in the step (c) is about 1;
more preferably, a mass ratio of the lubricant added in the step (a) to the lubricant added in the step (c) is about 2;
also preferably, conditions for the dry granulation are as follows: a hydraulic pressure of 18 bar to 50 bar, a roller rotation speed of 3.4 rpm to 14.8 rpm, a roller gap of 1 mm to 4 mm, a secondary granulation speed of 24.9 rpm to 108.4 rpm, and a primary granulation speed of 10 rpm to 90 rpm;
also preferably, a specification for a primary sizing screen in the dry granulation is 2.0 mm, and a specification for a secondary sizing screen in the dry granulation is 0.8 mm.

10. Use of the first pharmaceutical composition according to any one of claims 1 to 3 or the second pharmaceutical composition according to claim 4 in preparation of a medicament for modulating a protein kinase and/or for treating a protein kinase-mediated disease, disorder, and/or pathology;
preferably, the disease, disorder, and/or pathology is a cancer, a vascular disorder, a hypoxic disorder, osteoporosis, or a disease affecting cell motility, adhesion, and cell cycle progression;
more preferably, the cancer is leukemia, lung cancer, liver cancer, endometrial cancer, gastric cancer, rectal cancer, colon cancer, CNS cancer, melanoma, ovarian cancer, renal cancer, prostate cancer, or breast cancer;
more preferably, the vascular disorder is myocardial infarction, stroke, or ischemia.
